# EUROPEAN PATENT APPLICATION

(11) **EP 4 292 654 A2**
(43) Date of publication of application: **20.12.2023**
(21) Application number: 23189057.5
(22) Date of filing: 11.09.2015
(51) Int. Cl.: A61P 25/28

(54) **DETECTION OF MISFOLDED PROTEINS**

(30) Priority: 11.09.2014 US 201462049306 P
(62) Divisional of application: 15839278.7
(71) Applicant: Board of Regents of the University of Texas System, Austin, TX 78701 (US); Amprion, Inc., San Francisco, California 94105 (US); Jara, Claudio Soto, Houston, Texas 77025 (US); Shahnawaz, Mohammad, Houston, Texas 77005 (US); Lebovitz, Russell M., Oakland, California 94607 (US); Vollrath, Benedikt K., San Diego, California 92107 (US)
(72) Inventor: JARA, Claudio, Soto, Houston, TX 77025 (US); SHAHNAWAZ, Mohammad, Houston, TX 77025 (US); LEBOVITZ, Russell M., Oakland, CA 94607 (US); VOLLRATH, Benedikt, K., San Diego, CA 92107 (US)
(74) Representative: Forresters IP LLP

(57) **Abstract**

Methods and kits are provided for amplifying and detecting misfolded proteins from samples, for example, from patients having Alzheimer 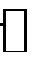 s Disease, Parkinson 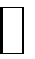 s Disease, and the like. For example, a method for determining a presence of soluble, misfolded protein in a sample may include contacting the sample with a monomeric, folded protein to form an incubation mixture; conducting an incubation cycle two or more times effective to form an amplified portion of misfolded protein; incubating the incubation mixture effective to cause misfolding and/or aggregation of at least a portion of the monomeric, folded protein; physically disrupting the incubation mixture effective to break up at least a portion of any protein aggregate present; and determining the presence of the soluble, misfolded protein in the sample by detecting at least a portion of the soluble, misfolded protein. The monomeric, folded protein and the soluble, misfolded protein may exclude prion protein (PrP) and isoforms thereof.

## Description

### CROSS-REFERENCE TO RELATED APPLICATIONS

This application claims priority from U.S. Provisional Patent Application No. 62/049,306, filed on September 11, 2014, the entire contents of which are incorporated herein by reference.

### BACKGROUND

Protein misfolding disorders (PMDs) include: Alzheimer's disease, Parkinson's disease, type 2 diabetes, Huntington's disease, amyotrophic lateral sclerosis, systemic amyloidosis, prion diseases, and the like. Misfolded aggregates of different proteins may be formed and accumulate. The misfolded aggregates may induce cellular dysfunction and tissue damage, among other effects.

For example, Alzheimer's disease (AD) and Parkinson's disease (PD) are degenerative brain disorders with no effective treatment or accurate preclinical diagnosis. In AD, for example, evidence to date suggests that the misfolding, aggregation, and brain deposition of the amyloid-beta protein (Aβ) may be triggering factors for AD pathology. While Aβ plaques were originally thought to be the hallmark of the disease, current research suggests that soluble Aβ oligomers may be critical synapto-toxic species causing neurodegeneration in AD. Because the brain has low regeneration capacity, early diagnosis of PMDs is crucial to permit intervention before irreversible neuropathological changes occur. Several lines of evidence indicate that the process of misfolding and oligomerization may begin years or decades before the onset of clinical symptoms and substantial brain damage. The lack of widely accepted early, sensitive, and objective laboratory diagnosis remains a major problem for care of patients suffering from PMDs.

The present application appreciates that detection of protein misfolding, e.g., for diagnosis of related diseases may be a challenging endeavor.

### SUMMARY

In one embodiment, a method for determining a presence of soluble, misfolded protein in a sample is provided. The method may include contacting the sample with a monomeric, folded protein to form an incubation mixture. The method may include conducting an incubation cycle two or more times effective to form an amplified portion of misfolded protein. Each incubation cycle may include incubating the incubation mixture effective to cause misfolding and/or aggregation of at least a portion of the monomeric, folded protein in the presence of the soluble, misfolded protein. Each incubation cycle may include physically disrupting the incubation mixture effective to break up at least a portion of any protein aggregate present, e.g., to release the soluble, misfolded protein. The method may include determining the presence of the soluble, misfolded protein in the sample by detecting at least a portion of the soluble, misfolded protein. The soluble, misfolded protein may include one or more of: a soluble, misfolded monomer and a soluble, misfolded aggregate. The amplified portion of misfolded protein may include one or more of: an amplified portion of the soluble, misfolded monomer, an amplified portion of the soluble, misfolded aggregate, and an insoluble, misfolded aggregate. The monomeric, folded protein and the soluble, misfolded protein may exclude prion protein (PrP) and isoforms thereof.

In another embodiment, a method for determining a presence of soluble, misfolded protein in a sample is provided. The method may include contacting the sample with Thioflavin T and a molar excess of a monomeric, folded protein to form an incubation mixture. The molar excess may be greater than an amount of protein monomer included in the soluble, misfolded protein in the sample. The method may include conducting an incubation cycle two or more times effective to form an amplified portion of misfolded protein. Each incubation cycle may include incubating the incubation mixture effective to cause misfolding and/or aggregation of at least a portion of the monomeric, folded protein in the presence of the soluble, misfolded protein to form the amplified portion of misfolded protein. Each incubation cycle may include shaking the incubation mixture effective to break up at least a portion of any protein aggregate present, e.g., to release the soluble, misfolded protein. The method may also include determining the presence of the soluble, misfolded protein in the sample by detecting a fluorescence of the Thioflavin T corresponding to soluble, misfolded protein. The soluble, misfolded protein may include one or more of: a soluble, misfolded monomer and a soluble, misfolded aggregate. The amplified portion of misfolded protein may include one or more of: an amplified portion of the soluble, misfolded monomer, an amplified portion of the soluble, misfolded aggregate, and an insoluble, misfolded aggregate. The monomeric, folded protein and the soluble, misfolded protein exclude prion protein (PrP) and isoforms thereof.

In one embodiment, a method for determining a presence of a soluble, misfolded protein in a sample is provided. The method may include capturing soluble, misfolded protein from the sample. The method may include contacting the captured soluble, misfolded protein with a molar excess of monomeric, folded protein to form an incubation mixture. The molar excess may be greater than an amount of protein monomer included in the captured soluble, misfolded protein. The method may include conducting an incubation cycle two or more times effective to form an amplified portion of misfolded protein. Each incubation cycle may include incubating the incubation mixture effective to cause misfolding and/or aggregation of at least a portion of the monomeric, folded protein in the presence of the captured soluble, misfolded protein to form the amplified portion of misfolded protein. Each incubation cycle may include physically disrupting the incubation mixture effective to break up at least a portion of any protein aggregate present, e.g., to release the soluble, misfolded protein. The method may also include determining the presence of the soluble, misfolded protein in the sample by detecting at least a portion of the soluble, misfolded protein. The soluble, misfolded protein may include one or more of: a soluble, misfolded monomer and a soluble, misfolded aggregate. The captured soluble, misfolded protein may include one or more of: a captured soluble, misfolded monomer and a captured soluble, misfolded aggregate. The amplified portion of misfolded protein may include one or more of: an amplified portion of the soluble, misfolded monomer, an amplified portion of the soluble, misfolded aggregate, and an insoluble, misfolded aggregate. The monomeric, folded protein and the soluble, misfolded protein exclude prion protein (PrP) and isoforms thereof.

In another embodiment, a kit for determining a presence of a soluble, misfolded protein in a sample is provided. The kit may include one or more of a known amount of a monomeric, folded protein and a known amount of an indicator of the soluble, misfolded protein. The kit may include instructions. The instructions may direct a user to contact the sample with one or more of the known amount of the monomeric, folded protein and the known amount of the indicator of the soluble, misfolded protein to form an incubation mixture. The instructions may direct a user to conduct an incubation cycle two or more times effective to form an amplified portion of misfolded protein. Each incubation cycle may include incubating the incubation mixture effective to cause misfolding and/or aggregation of at least a portion of the monomeric, folded protein in the presence of the soluble, misfolded protein to form the amplified portion of misfolded protein. Each incubation cycle may include physically disrupting the incubation mixture effective to break up at least a portion of any protein aggregate present, e.g., to release the soluble, misfolded protein. The instructions may direct a user to determine the presence of the soluble, misfolded protein in the sample by detecting the soluble, misfolded protein. The soluble, misfolded protein may include one or more of: a soluble, misfolded monomer and a soluble, misfolded aggregate. The amplified portion of misfolded protein may include one or more of: an amplified portion of the soluble, misfolded monomer, an amplified portion of the soluble, misfolded aggregate, and an insoluble, misfolded aggregate. The monomeric, folded protein and the soluble, misfolded protein may exclude prion protein (PrP) and isoforms thereof.

The methods and kits disclosed herein for determining a presence of a soluble, misfolded protein in a sample may be effective to determine an absence of the soluble, misfolded protein in the sample.

### BRIEF DESCRIPTION OF THE DRAWINGS

The accompanying figures, which are incorporated in and constitute a part of the specification, illustrate example methods and results, and are used merely to illustrate example embodiments.
**FIG. 1A** shows electron micrographs taken at 0h, 5h, 10h, and 24h of incubation.
**FIG. 1B** is a western blot of soluble oligomeric Aβ protein aggregates.
**FIG. 2A** is a graph showing non-amplified amyloid formation measured by ThT fluorescence as a function of time seeded by various concentrations of synthetic soluble oligomeric Aβ protein of **EXAMPLE 1.**
**FIG. 2B** is a graph showing amplification cycle-accelerated amyloid formation measured by ThT fluorescence as a function of time seeded by various concentrations of synthetic soluble oligomeric Aβ protein of **EXAMPLE 1.**
**FIG. 3A** is a graph of amyloid formation versus time, measured as a function of ThT fluorescence labeling, showing the average kinetics of Aβ aggregation seeded by CSF from 5 representative samples from the AD, NND, and NAND groups.
**FIG. 3B** is a graph of the lag phase time in h for Aβ aggregation in the presence of samples from the AD, NND, and NAND groups.
**FIG. 3C** is a graph showing the extent of amyloid formation obtained after 180 Aβ-PMCA cycles, i.e. 90 h of incubation (P90) in the presence of CSF samples from AD, NND and NAND patients.
**FIGS. 4A-D** are plots of the true positive rate (sensitivity) as a function of the false positive rate (specificity) for different cut-off points using the lag phase values showed in **FIG. 3B** for AD vs NAND (**FIG. 4A**), AD vs NND (**FIG. 4B**) and AD vs All control samples (**FIG. 4C**). **FIG. 4D** estimates the most reliable cut-off point for the different set of group comparisons.
**FIG. 5****, Table 1** shows estimations of the sensitivity, specificity and predictive value of the Aβ-PMCA test, calculated using the lag phase numbers.
**FIG. 6** is a graph of the lag phase time in h for samples obtained after 300 Aβ-PMCA cycles, i.e. 150 h of incubation (P90) in the presence of CSF samples from AD and control patients.
**FIG. 7A** is a western blot showing results of immunodepletion using synthetically prepared Aβ oligomers spiked into human CSF.
**FIG. 7B** is a graph showing the kinetics of Aβ aggregation seeded by control and immunodepleted CSF samples.
**FIG. 7C** is a graph showing the kinetics of Aβ aggregation seeded by control and immunodepleted CSF samples, depleted only with the A11 conformational antibody.
**FIG. 8A** is a schematic representation of an ELISA solid phase method employed to capture Aβ oligomers from complex biological samples.
**FIG. 8B** is a schematic representation of a magnetic bead solid phase method employed to capture Aβ oligomers from complex biological samples.
**FIG. 9****, Table 2** shows the ability of specific antibodies to capture the Aβ oligomers.
**FIG. 10** is a graph of amyloid formation versus time showing the acceleration of Aβ aggregation by the presence of different quantities of synthetic oligomers spiked in human plasma.
**FIG. 11** is a graph showing time to reach 50% aggregation in an Aβ-PMCA assay in the presence of plasma samples from AD patients and controls.
**FIG. 12** is a western blot showing the results of amplification of Aβ aggregation by cycles of incubation/sonication in the presence of distinct quantities of synthetic Aβ oligomers monitored by Western blot after protease digestion.
**FIG. 13A** is a graph of Thioflavin T fluorescence versus time showing the detection of αS seeds by PD-PMCA.
**FIG. 13B** is a graph of time to reach 50% aggregation plotted as a function of the indicated amounts αS seeds.
**FIG. 14** shows detection of αS seeds in CSF samples from human PD patients by PD-PMCA, versus controls with Alzheimer's disease (AD) or a non-neurodegenerative disease (NND).
**FIG. 15****, Table 3** demonstrates the ability of different sequence or conformational antibodies to capture αS oligomers.
**FIG. 16A** is a schematic representation of an ELISA solid phase method employed to capture αS oligomers.
**FIG. 16B** is a schematic representation of a magnetic bead solid phase method employed to capture αS oligomers.
**FIGS. 17A****,** **17B****,** and **17C** are a series of graphs that show the results of immunoprecipitation/aggregation of α-Synuclein oligomers from human blood plasma using three different α-Synuclein antibodies. **FIG. 17A** shows results with antibody N-19. **FIG. 17B** shows results with antibody 211. **FIG. 17C** shows results with antibody C-20.
**FIGS. 18A****,** **18B****,** and **18C** are a series of graphs that show the results of detection for αS seeds in CSF samples. **FIG. 18A** shows results in control samples. **FIG. 18B** shows results in PD patients. **FIG. 18C** shows results in patients with Multiple System Atrophy (MSA).

### DETAILED DESCRIPTION

Methods and kits are provided for the detection of misfolded proteins in a sample, including for the diagnosis of protein misfolding disorders (PMDs). Misfolded aggregates of different proteins may be formed and accumulate. The misfolded aggregates may induce cellular dysfunction and tissue damage, among other effects. For example, PMDs may include: amyloidosis such as Alzheimer's disease (AD) or systemic amyloidosis; synucleinopathies such as Parkinson's disease (PD), Lewy body dementia; multiple system atrophy; and synuclein-related neuroaxonal dystrophy; type 2 diabetes; triplet repeat disorders such as Huntington's disease (HD); amyotrophic lateral sclerosis (ALS); and the like. Misfolded aggregates of different proteins may be formed and accumulate. The misfolded aggregates may induce cellular dysfunction and tissue damage, among other effects.

In some embodiments, PMDs may exclude infectious prion diseases, e.g., transmissible spongiform encephalopathy diseases (TSE). Such transmissible spongiform encephalopathies (TSE) are a group of infectious neurodegenerative diseases that affect humans and animals. For example, human TSE diseases may include: Creutzfeldt-Jakob disease and its variant (CJD, vCJD), kuru, Gerstmann-Straussler-Scheiker disease (GSS), and fatal familial insomnia (FFI). Animal TSE diseases may include sheep and goats (scrapie); cattle (bovine spongiform encephalopathy, BSE); elk, white-tailed deer, mule deer and red deer (Chronic Wasting Disease, CWD); mink (transmissible mink encephalopathy, TME); cats (feline spongiform encephalopathy, FSE); nyala and greater kudu (exotic ungulate encephalopathy, EUE); and the like

As used herein, "monomeric protein" and "soluble, misfolded protein" exclude prion protein (PrP) and isoforms thereof. As used herein, a "prion" is a misfolded protein known to cause a TSE. Prion protein (PrP) may be associated with several isoforms: a normal, common, or cellular isoform (PrP^{C}); a protease resistant isoform (PrP^{res}); an infectious or "scrapie" isoform (PrP^{Sc}); and the like.

The methods, Protein Misfolding Cyclic Amplification (PMCA), may provide ultra-sensitive detection of misfolded aggregates through artificial acceleration and amplification of the misfolding and aggregation process *in vitro.* The basic concept of PMCA has been disclosed previously (Soto et al, WO 2002/04954; Estrada, et al. U.S. Pat. App. Pub. No. 20080118938, each of which is entirely incorporated herein by reference). However, prior to the filing date of the present document, no patent publication has enabled PCMA for the amplification and detection of soluble, misfolded protein other than prions in a sample, including for the diagnosis of PMDs. This document discloses specific examples and details which enable PMCA technology for the detection of soluble, misfolded protein, as may be found in PMD patients.

In various embodiments, methods for determining a presence of a soluble, misfolded protein in a sample are provided. As described herein, methods and kits for determining a presence of a soluble, misfolded protein in a sample may be effective to determine an absence of the soluble, misfolded protein in the sample. The soluble, misfolded protein described herein may be a pathogenic protein, e.g., causing or leading to various neural pathologies associated with PMDs. The methods may include contacting the sample with a monomeric, folded protein to form an incubation mixture. The methods may include conducting an incubation cycle two or more times effective to form an amplified portion of misfolded protein. Each incubation cycle may include incubating the incubation mixture effective to cause misfolding and/or aggregation of at least a portion of the monomeric, folded protein in the presence of the soluble, misfolded protein, e.g., to form an amplified portion of misfolded protein. Each incubation cycle may include physically disrupting the incubation mixture effective to break up at least a portion of any protein aggregate present e.g., to release the soluble, misfolded protein. The methods may include determining the presence of the soluble, misfolded protein in the sample by detecting at least a portion of the soluble, misfolded protein. The soluble, misfolded protein may include one or more of: a soluble, misfolded monomer and a soluble, misfolded aggregate. The amplified portion of misfolded protein may include one or more of: an amplified portion of the soluble, misfolded monomer, an amplified portion of the soluble, misfolded aggregate, and an insoluble, misfolded aggregate. The monomeric, folded protein and the soluble, misfolded protein may exclude prion protein (PrP) and isoforms thereof.

As used herein, "Aβ" or "beta amyloid" refers to a peptide formed via sequential cleavage of the amyloid precursor protein (APP). Various Aβ isoforms may include 38-43 amino acid residues. The Aβ protein may be formed when APP is processed by β- and/or γ-secretases in any combination. The Aβ may be a constituent of amyloid plaques in brains of individuals suffering from or suspected of having AD. Various Aβ isoforms may include and are not limited to Abeta40 and Abeta42. Various Aβ peptides may be associated with neuronal damage associated with AD.

As used herein, "αS" or "alpha-synuclein" refers to full-length, 140 amino acid α-synuclein protein, e.g., "αS-140." Other isoforms or fragments may include "αS-126," alpha-synuclein-126, which lacks residues 41-54, e.g., due to loss of exon 3; and "αS-112" alpha-synuclein-112, which lacks residue 103-130, e.g., due to loss of exon 5. The αS may be present in brains of individuals suffering from PD or suspected of having PD. Various αS isoforms may include and are not limited to αS-140, αS-126, and αS-112. Various αS peptides may be associated with neuronal damage associated with PD.

As used herein, "tau" refers to proteins are the product of alternative splicing from a single gene, e.g., MAPT (microtubule-associated protein tau) in humans. Tau proteins include to full-length and truncated forms of any of tau's isoforms. Various isoforms include, but are not limited to, the six tau isoforms known to exist in human brain tissue, which correspond to alternative splicing in exons 2, 3, and 10 of the tau gene. Three isoforms have three binding domains and the other three have four binding domains. Misfolded tau may be present in brains of individuals suffering from AD or suspected of having AD, or other tauopathies.

As used herein, a "misfolded protein" is a protein that no longer contains all or part of the structural conformation of the protein as it exists when involved in its typical, nonpathogenic normal function within a biological system. A misfolded protein may aggregate. A misfolded protein may localize in protein aggregate. A misfolded protein may be a non-functional protein. A misfolded protein may be a pathogenic conformer of the protein. Monomeric, folded protein compositions may be provided in native, nonpathogenic confirmations without the catalytic activity for misfolding, oligomerization, and aggregation associated with seeds. Monomeric, folded protein compositions may be provided in seed-free form.

As used herein, "monomeric, folded protein" refers to single protein molecules. "Soluble, aggregated misfolded protein" refers to aggregations of monomeric, misfolded protein that remain in solution. Examples of soluble, misfolded protein may include any number of protein monomers so long as the misfolded protein remains soluble. For example, soluble, misfolded protein may include monomers or aggregates of between 2 and about 50 units of monomeric protein.

Monomeric and/or soluble, misfolded protein may aggregate to form insoluble aggregates and/or higher oligomers. For example, aggregation of Aβ protein may lead to protofibrils, fibrils, and eventually amyloid plaques that may be observed in AD subjects. "Seeds" or "nuclei" refer to soluble, misfolded protein or short fragmented fibrils, particularly soluble, misfolded protein with catalytic activity for further misfolding, oligomerization, and aggregation. Such nucleation-dependent aggregation may be characterized by a slow lag phase wherein aggregate nuclei may form, which may then catalyze rapid formation of further aggregates and larger polymers. The lag phase may be minimized or removed by addition of pre-formed nuclei or seeds. Monomeric protein compositions may be provided without the catalytic activity for misfolding and aggregation associated with seeds. Monomeric protein compositions may be provided in seed-free form.

As used herein, "soluble" species may form a solution in biological fluids under physiological conditions, whereas "insoluble" species may be present as precipitates, fibrils, deposits, tangles, or other non-dissolved forms in such biological fluids under physiological conditions. Such biological fluids may include, for example, fluids, or fluids expressed from one or more of: amniotic fluid; bile; blood; cerebrospinal fluid; cerumen; skin; exudate; feces; gastric fluid; lymph; milk; mucus, e.g. nasal secretions; mucosal membrane, e.g., nasal mucosal membrane; peritoneal fluid; plasma; pleural fluid; pus; saliva; sebum; semen; sweat; synovial fluid; tears; urine; and the like. Insoluble species may include, for example, fibrils of Aβ, αS , tau, and the like. A species that dissolves in a nonbiological fluid but not one of the aforementioned biological fluids under physiological conditions may be considered insoluble. For example, fibrils of Aβ, αS, tau, and the like may be dissolved in a solution of, e.g., a surfactant such as sodium dodecyl sulfate (SDS) in water, but may still be insoluble in one or more of the mentioned biological fluids under physiological conditions.

In some embodiments, the sample may exclude insoluble species of the misfolded proteins such as Aβ, αS, or tau as a precipitate, fibril, deposit, tangle, plaque, or other form that may be insoluble in one or more of the described biological fluids under physiological conditions.

For example, the sample may exclude αS and tau in fibril form. The sample may exclude misfolded Aβ, αS and/or tau proteins in insoluble form, e.g., the sample may exclude the misfolded Aβ, αS and/or tau proteins as precipitates, fibrils, deposits, tangles, plaques, or other insoluble forms, e.g., in fibril form. The methods described herein may include preparing the sample by excluding the misfolded Aβ and tau proteins in insoluble form, e.g., by excluding from the sample the misfolded Aβ and tau proteins as precipitates, fibrils, deposits, tangles, plaques, or other insoluble forms, e.g., in fibril form. The kits described herein may include instructions directing a user to prepare the sample by excluding from the sample the misfolded Aβ, αS and/or tau proteins as precipitates, fibrils, deposits, tangles, plaques, or other insoluble forms, e.g., in fibril form. The exclusion of such insoluble forms of the described misfolded proteins from the sample may be substantial or complete.

As used herein, aggregates of soluble, misfolded protein refer to non-covalent associations of protein including soluble, misfolded protein. Aggregates of soluble, misfolded protein may be "de-aggregated", or disrupted to break up or release soluble, misfolded protein. The catalytic activity of a collection of soluble, misfolded protein seeds may scale, at least in part with the number of seeds in a mixture. Accordingly, disruption of aggregates of soluble, misfolded protein in a mixture to release soluble, misfolded protein seeds may lead to an increase in catalytic activity for oligomerization of monomeric protein.

As used herein, a "misfolded protein" is a protein that no longer contains all or part of the structural conformation of the protein as it exists when involved in its typical normal function within a biological system. A misfolded protein may aggregate. A misfolded protein may localize in protein aggregate. A misfolded protein may be a non-functional protein. A misfolded protein may be a pathological isoform.

In various embodiments, methods for determining a presence of a soluble, misfolded protein in a sample are provided. The methods may include contacting the sample with Thioflavin T and a molar excess of a monomeric, folded protein to form an incubation mixture. The molar excess may be greater than an amount of protein monomer included in the soluble, misfolded protein in the sample. The methods may include conducting an incubation cycle two or more times effective to form an amplified portion of misfolded protein. Each incubation cycle may include incubating the incubation mixture effective to cause misfolding and/or aggregation of at least a portion of the monomeric, folded protein in the presence of the soluble, misfolded protein to form the amplified portion of misfolded protein. Each incubation cycle may include shaking the incubation mixture effective to break up at least a portion of any protein aggregate present, e.g., to release the soluble, misfolded protein. The methods may also include determining the presence of the soluble, misfolded protein in the sample by detecting a fluorescence of the Thioflavin T corresponding to soluble, misfolded protein. The soluble, misfolded protein may include one or more of: a soluble, misfolded monomer and a soluble, misfolded aggregate. The captured soluble, misfolded protein may include one or more of: a captured soluble, misfolded monomer and a captured soluble, misfolded aggregate. The amplified portion of misfolded protein may include one or more of: an amplified portion of the soluble, misfolded monomer, an amplified portion of the soluble, misfolded aggregate, and an insoluble, misfolded aggregate. In some embodiments, the monomeric, folded protein and the soluble, misfolded protein may exclude prion protein (PrP) and isoforms thereof.

In various embodiments, methods for determining a presence of a soluble, misfolded protein in a sample are provided. The methods may include capturing soluble, misfolded protein from the sample. The methods may include contacting the captured soluble, misfolded protein with a molar excess of monomeric, folded protein to form an incubation mixture. The molar excess may be greater than an amount of protein monomer included in the captured soluble, misfolded protein. The methods may include conducting an incubation cycle two or more times effective to form an amplified portion of misfolded protein. Each incubation cycle may include incubating the incubation mixture effective to cause misfolding and/or aggregation of at least a portion of the monomeric, folded protein in the presence of the captured soluble, misfolded protein to form an amplified portion of misfolded protein. Each incubation cycle may include physically disrupting the incubation mixture effective to break up at least a portion of any protein aggregate present, e.g., to release the soluble, misfolded protein. The methods may also include determining the presence of the soluble, misfolded protein in the sample by detecting at least a portion of the soluble, misfolded protein. The soluble, misfolded protein may include one or more of: a soluble, misfolded monomer and a soluble, misfolded aggregate. The captured soluble, misfolded protein may include one or more of: a captured soluble, misfolded monomer and a captured soluble, misfolded aggregate. The amplified portion of misfolded protein may include one or more of: an amplified portion of the soluble, misfolded monomer, an amplified portion of the soluble, misfolded aggregate, and an insoluble, misfolded aggregate. In some embodiments, the monomeric, folded protein and the soluble, misfolded protein may exclude prion protein (PrP) and isoforms thereof.

As used herein, references to the soluble, misfolded protein may include any form of the soluble, misfolded protein, distributed in the sample, the incubation mixture, and the like. For example, references to the soluble, misfolded protein may include the soluble, misfolded protein, for example, the soluble, misfolded protein in a sample from a subject suffering from a PMD. References to the soluble, misfolded protein may include, for example, the amplified portion of misfolded protein, e.g., in the incubation mixture. References to the soluble, misfolded protein may include the captured soluble, misfolded protein, e.g., soluble, misfolded protein captured from the sample using soluble, misfolded protein-specific antibodies.

In some embodiments, the methods may include contacting an indicator of the soluble, misfolded protein to the incubation mixture. The indicator of the soluble, misfolded protein may be characterized by an indicating state in the presence of the soluble, misfolded protein and a non-indicating state in the absence of the soluble, misfolded protein. The determining the presence of the soluble, misfolded protein in the sample may include detecting the indicating state of the indicator of the soluble, misfolded protein. The indicating state of the indicator and the non-indicating state of the indicator may be characterized by a difference in fluorescence. The determining the presence of the soluble, misfolded protein in the sample may include detecting the difference in fluorescence.

In several embodiments, the method may include contacting a molar excess of the indicator of the soluble, misfolded protein to the incubation mixture. The molar excess may be greater than a total molar amount of protein monomer included in the monomeric, folded protein and the soluble, misfolded protein in the incubation mixture.

In various embodiments, the indicator of the soluble, misfolded protein may include one or more of: Thioflavin T, Congo Red, m-I-Stilbene, Chrysamine G, PIB, BF-227, X-34, TZDM, FDDNP, MeO-X-04, IMPY, NIAD-4, luminescent conjugated polythiophenes, a fusion with a fluorescent protein such as green fluorescent protein and yellow fluorescent protein, derivatives thereof, and the like.

In some embodiments, determining the presence of the soluble, misfolded protein in the sample may include determining an amount of the soluble, misfolded protein in the sample. The amount of the soluble, misfolded protein in the sample may be determined compared to a control sample. The amount of the soluble, misfolded protein in the sample may be detected with a sensitivity of at least about one or more of: 80%, 81%, 82%, 83%, 84%, 85%, 86%, 87%, 88%, 89%, 90%, 91%, 92%, 93%, 94%, 95%, 96%, 97%, 98%, 99%, and 100%. The amount of the soluble, misfolded protein in the sample detected may be less than about one or more of: 100 nmol, 10 nmol, 1 nmol, 100 pmol, 10 pmol, 1 pmol, 100 fmol, 10 fmol, 3 fmol, 1 fmol, 100 attomol, 10 attomol, and 1 attomol. The amount of the soluble, misfolded protein in the sample may be detected in a molar ratio to monomeric, folded protein comprised by the sample. The molar ratio may be less than about one or more of 1: 100, 1: 10,000, 1: 100,000, and 1: 1,000,000.

In various embodiments, the soluble, misfolded protein in the sample may be detected with a specificity of at least about one or more of: 80%, 81%, 82%, 83%, 84%, 85%, 86%, 87%, 88%, 89%, 90%, 91%, 92%, 93%, 94%, 95%, 96%, 97%, 98%, 99%, and 100%.

In some embodiments, the incubation mixture may include the monomeric, folded protein in a concentration, or in a concentration range, of one or more of: between about 1 nM and about 2 mM; between about 10 nM and about 200 µM; between about 100 nM and about 20 µM; or between about 1 µM and about 10 µM; and about 2 µM.

In several embodiments, the incubation mixture may include a buffer composition. The buffer composition may be effective to prepare or maintain the pH of the incubation mixture as described herein, e.g., between pH 5 and pH 9. The buffer composition may include one or more of: Tris-HCL, PBS, MES, PIPES, MOPS, BES, TES, and HEPES, and the like. The buffer concentration may be at a total concentration of between about 1 µm and about 1M. For example, the buffer may be Tris-HCL at a concentration of 0.1 M.

In various embodiments, the incubation mixture may include a salt composition. The salt composition may be effective to increase the ionic strength of the incubation mixture. The salt composition may include one or more of: NaCl, KCl, and the like. The incubation mixture may include the salt composition at a total concentration of between about 1 µm and about 500 mM.

In several embodiments, the incubation mixture may be characterized by, prepared with, or maintained at a pH value of or a pH range of one or more of: between about 5 and about 9; between about 6 and about 8.5; between about 7 and about 8; and about 7.4.

In some embodiments, the incubation mixture may be incubated at a temperature in °C of about one or more of: 4, 6, 8, 10, 12, 14, 16, 18, 20, 22, 24, 26, 28, 30, 32, 34, 35, 36, 37, 40, 45, 50, 55, and 60, e.g., about 22 °C, or a temperature range between any two of the preceding values, for example, one or more of: between about 4 °C and about 60 °C; between about 4 °C and about 35 °C; between about 8 °C and about 50 °C; between about 12 °C and about 40 °C; between about 18 °C and about 30 °C; between about 18 °C and about 26 °C; and the like.

In several embodiments, the detecting the soluble, misfolded protein may include one or more of: a Western Blot assay, a dot blot assay, an enzyme-linked immunosorbent assay (ELISA), a thioflavin T binding assay, a Congo Red binding assay, a sedimentation assay, electron microscopy, atomic force microscopy, surface plasmon resonance, and spectroscopy. The ELISA may include a two-sided sandwich ELISA. The spectroscopy may include one or more of: quasi-light scattering spectroscopy, multispectral ultraviolet spectroscopy, confocal dual-color fluorescence correlation spectroscopy, Fourier-transform infrared spectroscopy, capillary electrophoresis with spectroscopic detection, electron spin resonance spectroscopy, nuclear magnetic resonance spectroscopy, Fluorescence Resonance Energy Transfer (FRET) spectroscopy, and the like.

In various embodiments, the detecting the soluble, misfolded protein may include contacting the incubation mixture with a protease. The soluble, misfolded protein may be detected using anti-misfolded protein antibodies in one or more of: a Western Blot assay, a dot blot assay, and an ELISA.

In some embodiments, the method may include providing the monomeric, folded protein in labeled form. The monomeric, folded protein in labeled form may include one or more of: a covalently incorporated radioactive amino acid, a covalently incorporated, isotopically labeled amino acid, and a covalently incorporated fluorophore. The detecting the soluble, misfolded protein include detecting the monomeric, folded protein in labeled form as incorporated into the amplified portion of misfolded protein.

In several embodiments, the sample may be taken from a subject. The method may include determining or diagnosing the presence of a PMD in the subject according to the presence of the soluble, misfolded protein in the sample. The presence of the soluble, misfolded protein in the sample may be determined compared to a control sample taken from a control subject.

In some embodiments, the PMD may include at least one of: an amyloidosis; a synucleinopathy; a triplet repeat disorder; or amyotrophic lateral sclerosis. The PMD may include amyloidosis, e.g., at least one of: Alzheimer's disease or systemic amyloidosis. The PMD may include a synucleinopathy, e.g., at least one of: Parkinson's disease, Lewy body dementia; multiple system atrophy; or synuclein-related neuroaxonal dystrophy. The PMD may include Huntington's disease.

In various embodiments, the detecting may include detecting an amount of the soluble, misfolded protein in the sample. The method may include determining or diagnosing the presence of a PMD in the subject by comparing the amount of the soluble, misfolded protein in the sample to a predetermined threshold amount.

In several embodiments, the sample may be taken from a subject exhibiting no clinical signs of dementia according to cognitive testing. The method may include determining or diagnosing the presence of a PMD in the subject according to the presence of the soluble, misfolded protein in the sample.

In some embodiments, the sample may be taken from a subject exhibiting no cortex plaques or tangles according to amyloid beta contrast imaging. The method may further include determining or diagnosing the presence of a PMD in the subject according to the presence of the soluble, misfolded protein in the sample.

In various embodiments, the sample may be taken from a subject exhibiting clinical signs of dementia according to cognitive testing. The method may further include determining or diagnosing the presence of a PMD as a contributing factor to the clinical signs of dementia in the subject according to the presence of the soluble, misfolded protein in the sample.

In some embodiments, the sample may include one or more of: amniotic fluid; bile; blood; cerebrospinal fluid; cerumen; skin; exudate; feces; gastric fluid; lymph; milk; mucus, e.g. nasal secretions; mucosal membrane, e.g., nasal mucosal membrane; peritoneal fluid; plasma; pleural fluid; pus; saliva; sebum; semen; sweat; synovial fluid; tears; urine; and the like.

In several embodiments, the method may include taking the sample from the subject. The subject may be one of a: human, mouse, rat, dog, cat, cattle, horse, deer, elk, sheep, goat, pig, or non-human primate. Non-human animals may be wild or domesticated. The subject may be one or more of: at risk of a PMD, having the PMD, under treatment for the PMD, at risk of having a disease associated with dysregulation, misfolding, aggregation or disposition of misfolding protein, having a disease associated with dysregulation, misfolding, aggregation or disposition of misfolding protein, under treatment for a disease associated with dysregulation, misfolding, aggregation or disposition of misfolding protein, and the like.

In various embodiments, the method may include determining or diagnosing a progression or homeostasis of a PMD in the subject by comparing the amount of the soluble, misfolded protein in the sample to an amount of the soluble, misfolded protein in a comparison sample taken from the subject at a different time compared to the sample. In various embodiments, the sample may be taken from a patient undergoing therapy for a PMD. A PMCA assay may be employed to determine which patients may be treated with a therapy.

For example, several novel therapeutics that are targeting Aβ homeostasis through various mechanisms are currently under development. A PMCA assay for Aβ oligomers may be employed to determine which patients may be treated with a PMD disease-modifying therapy. Patients showing a change, e.g., decrease or increase, in the level of Aβ oligomers as detected by the PMCA method may be classified as "responders" to Aβ modulating therapy, and may be treated with a therapeutic reducing the levels of Aβ oligomers. Patients lacking an aberrant Aβ homeostasis may be classified as "non responders" and may not be treated. Patients who could benefit from therapies aimed at modulating Aβ homeostasis may thus be identified.

Also, for example, several novel therapeutics that are targeting αS homeostasis through various mechanisms are currently under development. Therapeutic approaches targeting αS homeostasis may include active immunization, such as PD01A+ or PD03A+, or passive immunization such as PRX002. A PMCA assay for αS oligomers may be employed to determine which patients may be treated with an αS modulating therapy. Patients showing a change, e.g, increase or decrease, in the level of αS oligomers as detected by the PMCA method may be classified as "responders" to αS modulating therapy, and may be treated with a therapeutic reducing the levels of αS oligomers. Patients lacking an aberrant αS homeostasis may be classified as "non responders" and may not be treated. Patients who could benefit from therapies aimed at modulating αS homeostasis may thus be identified.

Further, for example, the amount of soluble, misfolded protein may be measured in samples from patients using PMCA. Patients with elevated soluble, misfolded protein measurements may be treated with therapeutics modulating soluble, misfolded protein homeostasis. Patients with normal soluble, misfolded protein measurements may not be treated. A response of a patient to therapies aimed at modulating soluble, misfolded protein homeostasis may be followed. For example, soluble, misfolded protein levels may be measured in a patient sample at the beginning of a therapeutic intervention. Following treatment of the patient for a clinical meaningful period of time, another patient sample may be obtained and soluble, misfolded protein levels may be measured. Patients who show a change in soluble, misfolded protein levels following therapeutic intervention may be considered to respond to the treatment. Patients who show unchanged soluble, misfolded protein levels may be considered non-responding. The methods may include detection of soluble, misfolded protein aggregates in patient samples containing components that may interfere with the PMCA reaction.

In some embodiments, the subject may be treated with a PMD modulating therapy. The method may include comparing the amount of the soluble, misfolded protein in the sample to an amount of the soluble, misfolded protein in a comparison sample. The sample and the comparison sample may be taken from the subject at different times over a period of time under the soluble, misfolded protein modulating therapy. The method may include determining or diagnosing the subject is one of: responsive to the soluble, misfolded protein modulating therapy according to a change in the soluble, misfolded protein over the period of time, or non-responsive to the soluble, misfolded protein modulating therapy according to homeostasis of the soluble, misfolded protein over the period of time. The method may include treating the subject determined to be responsive to the soluble, misfolded protein modulating therapy with the soluble, misfolded protein modulating therapy. For AD, for example, the PMD modulating therapy may include administration of one or more of: an inhibitor of BACE1 (beta-secretase 1); an inhibitor of γ-secretase; and a modulator of Aβ homeostasis, e.g., an immunotherapeutic modulator of Aβ homeostasis. The Aβ modulating therapy may include administration of one or more of: E2609; MK-8931; LY2886721; AZD3293; semagacestat (LY-450139); avagacestat (BMS-708163); solanezumab; crenezumab; bapineuzumab; BIIB037; CAD106; 8F5 or 5598 or other antibodies raised against Aβ globulomers, e.g., as described by Barghorn et al, "Globular amyloid β-peptide1-42 oligomer--a homogenous and stable neuropathological protein in Alzheimer's disease" J. Neurochem., 2005, 95, 834-847, the entire teachings of which are incorporated herein by reference; ACC-001; V950; Affitrope AD02; and the like.

For PD, for example, the PMD modulating therapy may include active immunization, such as PD01A+ or PD03A+, passive immunization such as PRX002, and the like. The PMD modulating therapy may also include treatment with GDNF (Glia cell-line derived neurotrophic factor), inosine, Calcium-channel blockers, specifically Cav1.3 channel blockers such as isradipine, nicotine and nicotine-receptor agonists, GM-CSF, glutathione, PPAR-gamma agonists such as pioglitazone, and dopamine receptor agonists, including D2/D3 dopamine receptor agonists and LRRK2 (leucine-rich repeat kinase 2) inhibitors.

In several embodiments, the amount of misfolded protein may be measured in samples from patients using PMCA. Patients with elevated misfolded protein measurements may be treated with disease modifying therapies for a PMD. Patients with normal misfolded protein measurements may not be treated. A response of a patient to disease-modifying therapies may be followed. For example, misfolded protein levels may be measured in a patient sample at the beginning of a therapeutic intervention. Following treatment of the patient for a clinical meaningful period of time, another patient sample may be obtained and misfolded protein levels may be measured. Patients who show a change in misfolded protein levels following therapeutic intervention may be considered to respond to the treatment. Patients who show unchanged misfolded protein levels may be considered non-responding. The methods may include detection of misfolded protein aggregates in patient samples containing components that may interfere with the PMCA reaction.

In several embodiments, the method may include selectively concentrating the soluble, misfolded protein in one or more of the sample and the incubation mixture. The selectively concentrating the soluble, misfolded protein may include pre-treating the sample prior to forming the incubation mixture. The selectively concentrating the soluble, misfolded protein may include pre-treating the incubation mixture prior to incubating the incubation mixture. The selectively concentrating the soluble, misfolded protein may include contacting one or more specific antibodies to the soluble, misfolded protein to form a captured soluble, misfolded protein.

For example, for AD, the one or more soluble, misfolded protein specific antibodies may include one or more of: 6E10, 4G8, 82E1, A11, X-40/42, 16ADV; and the like. Such antibodies may be obtained as follows: 6E10 and 4G8 (Covance, Princeton, NJ); 82E1 (IBL America, Minneapolis, MN); A11 (Invitrogen, Carlsbad, CA); X-40/42 (MyBioSource, Inc., San Diego, CA); and 16ADV (Acumen Pharmaceuticals, Livermore, CA).

Further, for PD, for example, the one or more soluble, misfolded protein specific antibodies may include PD specific antibodies including one or more of: α/β-synuclein N-19; α-synuclein C-20-R; α-synuclein 211; α-synuclein Syn 204; α-synuclein 2B2D1; α-synuclein LB 509; α-synuclein SPM451; α-synuclein 3G282; α-synuclein 3H2897; α/β-synuclein Syn 202; α/β-synuclein 3B6; α/β/γ-synuclein FL-140; and the like. In some examples, the one or more soluble, misfolded protein specific antibodies may include one or more of: α/β-synuclein N-19; α-synuclein C-20-R; α-synuclein 211; α-synuclein Syn 204; and the like. Such antibodies may be obtained as follows: α/β-synuclein N-19 (cat. No. SC-7012, Santa Cruz Biotech, Dallas, TX); α-synuclein C-20-R (SC-7011-R); α-synuclein 211 (SC-12767); α-synuclein Syn 204 (SC-32280); α-synuclein 2B2D1 (SC-53955); α-synuclein LB 509 (SC-58480); α-synuclein SPM451 (SC-52979); α-synuclein 3G282 (SC-69978); α-synuclein 3H2897 (SC-69977); α/β-synuclein Syn 202 (SC-32281); α/β-synuclein 3B6 (SC-69699); or α/β/γ-synuclein FL-140 (SC-10717).

Further, the one or more soluble, misfolded protein specific antibodies may include one or more of: an antibody specific for an amino acid sequence of the soluble, misfolded protein or an antibody specific for a conformation of the soluble, misfolded protein. The one or more soluble, misfolded protein specific antibodies may be coupled to a solid phase. The solid phase may include one or more of a magnetic bead and a multiwell plate.

For example, ELISA plates may be coated with the antibodies used to capture soluble, misfolded protein from the patient sample. The antibody-coated ELISA plates may be incubated with a patient sample, unbound materials may be washed off, and the PMCA reaction may be performed. Antibodies may also be coupled to beads. The beads may be incubated with the patient sample and used to separate soluble, misfolded protein -antibody complexes from the remainder of the patient sample.

In various embodiments, the contacting the sample with the monomeric, folded protein to form the incubation mixture may include contacting a molar excess of the monomeric, folded protein to the sample including the captured soluble, misfolded protein. The molar excess of the monomeric, folded protein may be greater than a total molar amount of protein monomer included in the captured soluble, misfolded protein. The incubating the incubation mixture may be effective to cause misfolding and/or aggregation of at least a portion of the monomeric, folded protein in the presence of the captured soluble, misfolded protein to form the amplified portion of misfolded protein.

In some embodiments, the protein aggregate may include one or more of: the monomeric protein, the soluble, misfolded protein, and a captured form of the soluble, misfolded protein.

In several embodiments, the physically disrupting the incubation mixture may include one or more of: sonication, stirring, shaking, freezing/thawing, laser irradiation, autoclave incubation, high pressure, homogenization, and the like. For example, shaking may include cyclic agitation. The cyclic agitation may be conducted between about 50 rotations per minute (RPM) and 10,000 RPM. The cyclic agitation may be conducted between about 200 RPM and about 2000 RPM. The cyclic agitation may be conducted at about 500 RPM.

In various embodiments, the physically disrupting the incubation mixture may be conducted in each incubation cycle for between about 5 seconds and about 10 minutes, between about 30 sec and about 1 minute, between about 45 sec and about 1 minute, for about 1 minute, and the like. For example, the physically disrupting the incubation mixture may be conducted in each incubation cycle by shaking for one or more of: between about 5 seconds and about 10 minutes, between about 30 sec and about 1 minute, between about 45 sec and about 1 minute, for about 1 minute, and the like. The incubating the incubation mixture may be independently conducted, in each incubation cycle, for a time between about 5 minutes and about 5 hours, between about 10 minutes and about 2 hours, between about 15 minutes and about 1 hour, between about 25 minutes and about 45 minutes, and the like. Each incubation cycle may include independently incubating and physically disrupting the incubation mixture for one or more of: incubating between about 5 minutes and about 5 hours and physically disrupting between about 5 seconds and about 10 minutes; incubating between about 10 minutes and about 2 hours and physically disrupting between about 30 sec and about 1 minute; incubating between about 15 minutes and about 1 hour and physically disrupting between about 45 sec and about 1 minute; incubating between about 25 minutes and about 45 minutes and physically disrupting between about 45 sec and about 1 minute; and incubating about 1 minute and physically disrupting about 1 minute.

The conducting the incubation cycle may be repeated between about 2 times and about 1000 times, between about 5 times and about 500 times, between about 50 times and about 500 times, between about 150 times and about 250 times, and the like. The incubating the incubation mixture being independently conducted, in each incubation cycle, at a temperature in °C of about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or a range between any two of the preceding values, for example, between about 15 °C and about 50 °C.

In several embodiments, contacting the sample with the monomeric, folded protein to form the incubation mixture may be conducted under physiological conditions. Contacting the sample with the monomeric, folded protein to form the incubation mixture may include contacting the sample with a molar excess of the monomeric protein. The molar excess may be greater than a total molar amount of protein monomer included in the soluble, misfolded protein in the sample. The monomeric, folded protein and/or the soluble, misfolded protein may include one or more peptides, e.g., formed by proteolytic cleavage of the monomeric, folded protein and/or the soluble, misfolded protein. For example, for AD, the monomeric, folded protein and/or the soluble, misfolded protein may include one or more peptides formed via β- or γ-secretase cleavage of amyloid precursor protein. For example, for AD, the monomeric, folded protein and/or the soluble, misfolded protein may include one or more of: Abeta40 and Abeta42. Further, for example, for PD, monomeric, folded protein and/or the soluble, misfolded protein may include one or more peptides formed via proteolytic cleavage of αS-140. The monomeric αS protein and/or the soluble oligomeric αS protein may include one or more of: αS-140, αS-126, αS-112, and the like. As used herein, "αS-140" refers to full-length, 140 amino acid α-synuclein protein. Other isoforms may include "αS-126," alpha-synuclein-126, which lacks residues 41-54, e.g., due to loss of exon 3; and "αS-112" alpha-synuclein-112, which lacks residue 103-130, e.g., due to loss of exon 5.

In various embodiments, the monomeric, folded protein may be produced by one of: chemical synthesis, recombinant production, or extraction from non-recombinant biological samples. The soluble, misfolded protein may substantially be the soluble, misfolded aggregate. The amplified portion of misfolded protein substantially being one or more of: the amplified portion of the soluble, misfolded aggregate and the insoluble, misfolded aggregate.

In various embodiments, kits for determining a presence of a soluble, misfolded protein in a sample are provided. The kits may include one or more of a known amount of a monomeric, folded protein and a known amount of an indicator of the soluble, misfolded protein. The kits may include instructions. The instructions may direct a user to conduct any of the methods described herein. For example, the instructions may direct the user to contact the sample with one or more of the known amount of the monomeric, folded protein and the known amount of the indicator of the soluble, misfolded protein to form an incubation mixture. The instructions may direct a user to conduct an incubation cycle two or more times effective to form an amplified portion of misfolded protein. Each incubation cycle may include incubating the incubation mixture effective to cause misfolding and/or aggregation of at least a portion of the monomeric, folded protein in the presence of the soluble, misfolded protein to form the amplified portion of misfolded protein. Each incubation cycle may include physically disrupting the incubation mixture effective to break up at least a portion of any protein aggregate present, e.g., to release the soluble, misfolded protein. The instructions may direct a user to determine the presence of the soluble, misfolded protein in the sample by detecting the soluble, misfolded protein. The soluble, misfolded protein may include the soluble, misfolded protein and the amplified portion of misfolded protein.

In various embodiments, the kit may include the known amount of the monomeric, folded protein and the known amount of the indicator of the soluble, misfolded protein. The kit may include one or more of: a multiwall microtitre plate; a microfluidic plate; a shaking apparatus; an incubating apparatus; and a fluorescence measurement apparatus; included either as one or more of the individual plates or apparatuses, as a combination device, and the like. For example, a shaking microplate reader may be used to perform cycles of incubation and shaking and automatically measure the ThT fluorescence emission during the course of an experiment (e.g., FLUOstar OPTIMA, BMG LABTECH Inc., Cary, NC).

In several embodiments of the kit, an indicating state and a non-indicating state of the indicator of the soluble, misfolded protein may be characterized by a difference in fluorescence. The instructions may direct the user to determine the presence of the soluble, misfolded protein in the sample by fluorescence spectroscopy.

In some embodiments of the kit, the indicator of the soluble, misfolded protein may include one or more of: Thioflavin T, Congo Red, m-I-Stilbene, Chrysamine G, PIB, BF-227, X-34, TZDM, FDDNP, MeO-X-04, IMPY, NIAD-4, luminescent conjugated polythiophenes, a fusion with a fluorescent protein such as green fluorescent protein and yellow fluorescent protein, derivatives thereof, and the like. The monomeric, folded protein may include one or more of a covalently incorporated radioactive amino acid, a covalently incorporated, isotopically labeled amino acid, a covalently incorporated fluorophore, and the like.

In various embodiments of the kit, the instructions may direct a user to conduct any of the methods described herein. For example, the instructions may include directions to the user to determine an amount of the soluble, misfolded protein in the sample. The instructions may direct the user to detect the soluble, misfolded protein by conducting one or more of: a Western Blot assay, a dot blot assay, an enzyme-linked immunosorbent assay (ELISA), a thioflavin T binding assay, a Congo Red binding assay, a sedimentation assay, electron microscopy, atomic force microscopy, surface plasmon resonance, spectroscopy, and the like.

The instructions may direct the user to detect the soluble, misfolded protein by contacting the incubation mixture with a protease; and detecting the soluble, misfolded protein using anti-misfolded protein antibodies in one or more of: a Western Blot assay, a dot blot assay, and an ELISA.

In several embodiments of the kit, the instructions may direct the user to take the sample from a subject. The instructions may include directing the user to determine the presence of a PMD in the subject according to the presence of the soluble, misfolded protein in the sample. The presence of the soluble, misfolded protein in the sample may be determined compared to a control sample taken from a control subject. The instructions may direct the user to determine the presence of the PMD in the subject by comparing the amount of the soluble, misfolded protein in the sample to a predetermined threshold amount. The instructions may direct the user to obtain the sample including one or more of: amniotic fluid; bile; blood; cerebrospinal fluid; cerumen; skin; exudate; feces; gastric fluid; lymph; milk; mucus, e.g. nasal secretions; mucosal membrane, e.g., nasal mucosal membrane; peritoneal fluid; plasma; pleural fluid; pus; saliva; sebum; semen; sweat; synovial fluid; tears; urine; and the like. The instructions may direct the user to determine a progression or homeostasis of the PMD in the subject by comparing the amount of the soluble, misfolded protein in the sample to an amount of the soluble, misfolded protein in a comparison sample taken from the subject at a different time compared to the sample.

The instructions may direct the user to the user to selectively concentrate the soluble, misfolded protein in one or more of the sample and the incubation mixture. For example, the kit may include one or more soluble, misfolded protein specific antibodies configured to selectively concentrate or capture the soluble, misfolded protein. The one or more soluble, misfolded protein specific antibodies may include one or more of: an antibody specific for an amino acid sequence of the soluble, misfolded protein and an antibody specific for a conformation of the soluble, misfolded protein. The instructions may direct the user to selectively concentrate the soluble, misfolded protein by contacting the one or more soluble, misfolded protein specific antibodies to the soluble, misfolded protein to form a captured soluble, misfolded protein. The one or more soluble, misfolded protein specific antibodies may be provided coupled to a solid phase. The solid phase may include one or more of a magnetic bead and a multiwell plate.

In various embodiments of the kit, the instructions for physically disrupting the incubation mixture may direct the user to employ one or more of: sonication, stirring, shaking, freezing/thawing, laser irradiation, autoclave incubation, high pressure, homogenization, and the like. The instructions may direct the user to conduct cyclic agitation according to any RPM range described herein, for example, between about 50 RPM and 10,000 RPM. The instructions may direct the user to conduct the physical disruption in each incubation cycle according to any time range described herein, for example, between about 5 seconds and about 10 minutes. The instructions may direct the user to incubate the incubation mixture in each incubation cycle according to any time range described herein, for example, for a time between about 5 minutes and about 5 hours. The instructions for conducting the incubation cycle may direct the user to conduct the incubation cycle for any number of repetitions described herein, for example, between about 2 times and about 1000 times. Instructions for conducting the incubation cycle may include directions to a user to incubate at a temperature between about 15 °C and about 50 °C.

In various embodiments of the kit, the monomeric, folded protein may be produced by one of: chemical synthesis, recombinant production, or extraction from non-recombinant biological samples. The soluble, misfolded protein may substantially be the soluble, misfolded aggregate. The amplified portion of misfolded protein substantially being one or more of: the amplified portion of the soluble, misfolded aggregate and the insoluble, misfolded aggregate.

### EXAMPLES

### EXAMPLE 1: PREPARATION OF SYNTHETIC Aβ OLIGOMERS

Aβ1-42 was synthesized using solid-phase N-tert-butyloxycarbonyl chemistry at the W. Keck Facility at Yale University and purified by reverse-phase HPLC. The final product was lyophilized and characterized by amino acid analysis and mass spectrometry. To prepare stock solutions free of aggregated, misfolded Aβ protein, aggregates were dissolved high pH and filtration through 30 kDa cut-off filters to remove remaining aggregates. To prepare different types of aggregates, solutions of seed-free Aβ1-42 (10 µM) were incubated for different times at 25 °C in 0.1 M Tris-HCl, pH 7.4 with agitation. This preparation contained a mixture of Aβ monomers as well as fibrils, protofibrils and soluble, misfolded Aβ protein in distinct proportions depending on the incubation time. The degree of aggregation was characterized by ThT fluorescence emission, electron microscopy after negative staining, dot blot studies with the A11 conformational antibody and western blot after gel electrophoresis using the 4G8 anti-Aβ antibody.

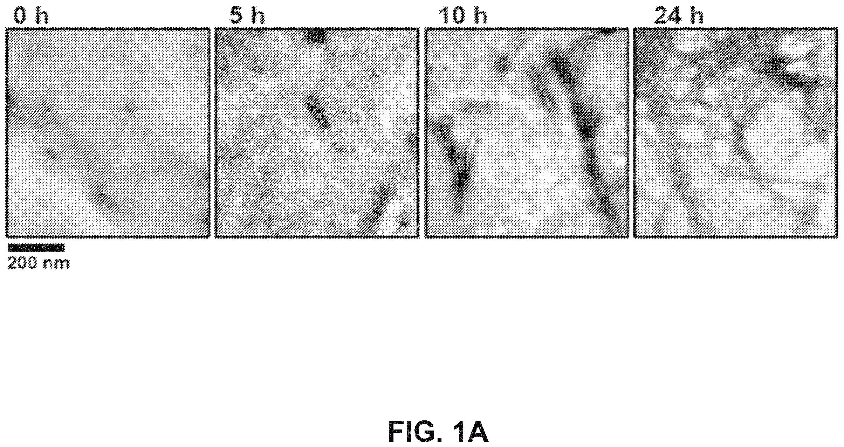

A mixture of Aβ oligomers of different sizes were generated during the process of fibril formation. Specifically, soluble, misfolded Aβ protein was prepared by incubation of monomeric synthetic Aβ1-42 (10 µM) at 25 °C with stirring. After 5 h of incubation, an abundance of soluble, misfolded Aβ protein, globular in appearance, was observed by electron microscopy after negative staining, with only a small amount of protofibrils and fibrils observed. At 10 h there are mostly protofibrils and at 24 h, a large amount of long fibrils are observed. **FIG. 1A** shows electron micrographs taken at 0h, 5h, 10h, and 24h of incubation.

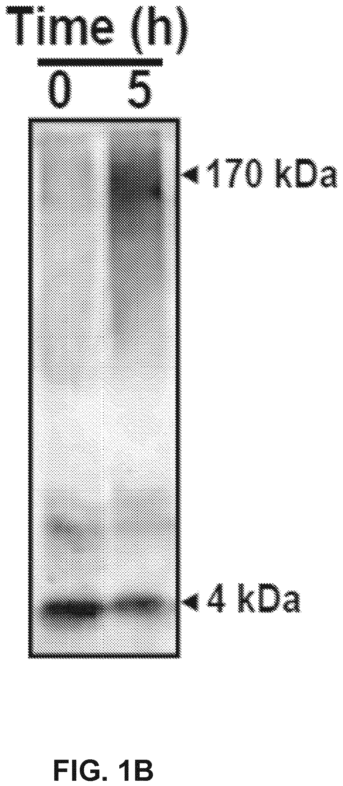

The soluble, misfolded Aβ protein aggregates tested positive using A11 anti-oligomer specific antibody according to the method of Kayed, et al. "Common structure of soluble amyloid oligomers implies common mechanism of pathogenesis," Science 2003, 300, 486-489. After further incubation at 10 h and 24 h, protofibrillar and fibrillar structures were observed. The size of the aggregates was determined by filtration through filters of defined pore size and western blotting after SDS-PAGE separation. Soluble, misfolded Aβ protein formed by incubation for 5 h was retained in filters of 30 kDa cut-off and passed through 1000 kDa cutoff filters. **FIG. 1B** is a western blot of soluble, misfolded Aβ protein aggregates. Electrophoretic separation of this soluble, misfolded Aβ protein showed that the majority of the material migrated as ~170 kDa SDS-resistant aggregates, with a minor band at 17 kDa.

### EXAMPLE 2: Aβ-PMCA DETECTS SYNTHETIC Aβ OLIGOMERS

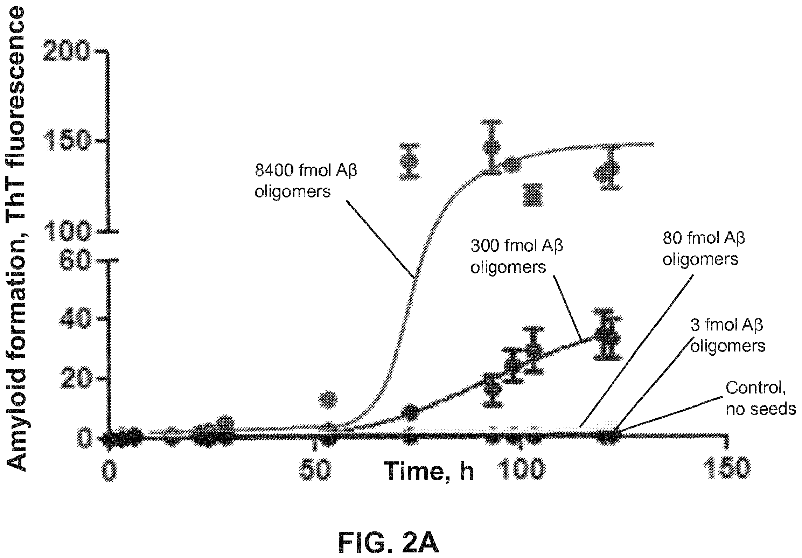

**EXAMPLE 2A**. Seeding of Aβ aggregation was studied by incubating a solution of seed-free Aβ1-42 in the presence of Thioflavin T with or without different quantities of synthetic soluble, misfolded Aβ protein (Control (no Aβ oligomer); or 3, 80, 300, and 8400 femtomolar in synthetic soluble, misfolded Aβ protein ). AP-PMCA general procedure: Solutions of 2 µM aggregate-free Aβ1-42 in 0.1 M Tris-HCl pH 7.4 (200 µL total volume) were placed in opaque 96-wells plates and incubated alone or in the presence of synthetic Aβ aggregates (prepared by incubation over 5h as described in **EXAMPLE 1**) or 40 µL of CSF aliquots. Samples were incubated in the presence of 5 µM Thioflavin T (ThT) and subjected to cyclic agitation (1 min at 500 rpm followed by 29 min without shaking) using an Eppendorf thermomixer, at a constant temperature of 22 °C. At various time points, ThT fluorescence was measured in the plates at 485 nm after excitation at 435 nm using a plate spectrofluorometer. **FIG. 2A** is a graph of amyloid formation (without cyclic amplification) versus time as measured by Thioflavin T fluorescence, using the indicated femtomolar concentration of synthetic soluble, misfolded Aβ protein seeds. The peptide concentration, temperature and pH of the buffer were monitored to control the extent of the lag phase and reproducibility among experiments. Under these conditions, no spontaneous Aβ aggregation was detected during the time in which the experiment was performed (125 h). Aggregation of monomeric Aβ1-42 protein was observed in the presence of 0.3 to 8.4 fmol of the synthetic soluble, misfolded Aβ protein of **EXAMPLE 1.**

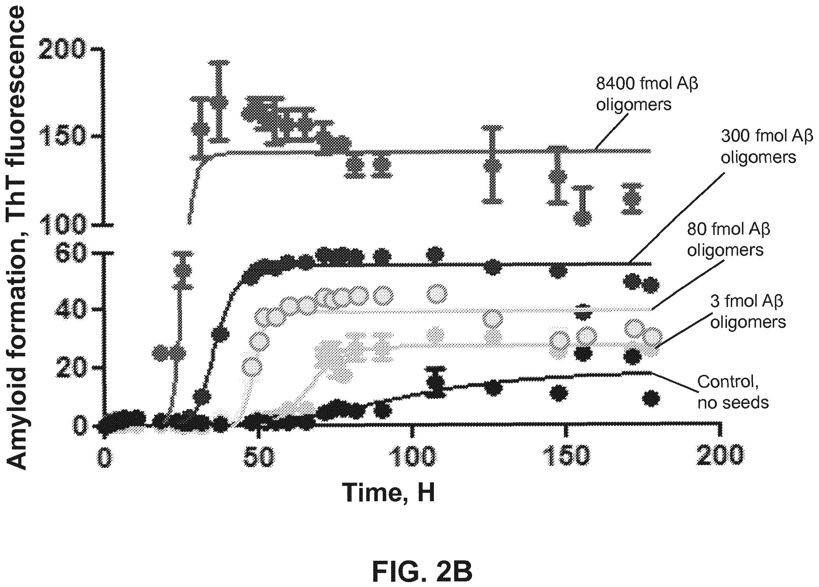

**EXAMPLE 2B:** Amplification cycles, combining phases of incubation and physical disruption were employed. The same samples as in **FIG. 2A** were incubated with cyclic agitation (1 min stirring at 500 rpm followed by 29 min without shaking). Aggregation was measured over time by the thioflavin T (ThT) binding to amyloid fibrils using a plate spectrofluorometer (excitation: 435; emission: 485 nm). Graphs show the mean and standard error of 3 replicates. The concentration of Aβ oligomers was estimated assuming an average molecular weight of 170 kDa. **FIG. 2B** is a graph showing amplification cycle-accelerated amyloid formation measured by ThT fluorescence as a function of time for various concentrations of the synthetic soluble, misfolded Aβ protein of **EXAMPLE 1.** Under these conditions, the aggregation of monomeric Aβ1-42 protein induced by 8400, 300, 80 and 3 fmol of the synthetic soluble, misfolded Aβ protein was clearly faster and more easily distinguished from that observed in the absence of the synthetic soluble, misfolded Aβ protein. This result indicates the detection limit, under these conditions, is 3 fmol of soluble, misfolded Aβ protein or less in a given sample.

### EXAMPLE 3: Aβ-PMCA DETECTS MISFOLDED Aβ IN THE CEREBROSPINAL FLUID OF AD PATIENTS

Aliquots of CSF were obtained from 50 AD patients, 39 cognitively normal individuals affected by non-degenerative neurological diseases (NND), and 37 patients affected by non-AD neurodegenerative diseases including other forms of dementia (NAND). Test CSF samples were obtained from 50 patients with the diagnosis of probable AD as defined by the DSM-IV and the NINCDS-ADRA guidelines (McKhann et al., 1984) and determined using a variety of tests, including routine medical examination, neurological evaluation, neuropsychological assessment, magnetic resonance imaging and measurements of CSF levels of Aβ1-42, total Tau and phospho-Tau. The mean age of AD patients at the time of sample collection was 71.0 ± 8.1 years (range 49-84). Control CSF samples were obtained from 39 patients affected by non-degenerative neurological diseases (NND), including 12 cases of normal pressure hydrocephalus, 7 patients with peripheral neuropathy, 7 with diverse forms of brain tumor, 2 with ICTUS, 1 with severe cephalgia, 3 with encephalitis, 1 with hypertension and 6 with unclear diagnosis. The mean age at which CSF samples were taken from this group of patients was 64.6 ± 14.7 years (range 31-83). Control CSF samples were also taken from 37 individuals affected by non-AD neurodegenerative diseases (NAND), including 10 cases of fronto-temporal dementia (5 behavioral and 5 language variants), 6 patients with Parkinson's disease (including 4 associated with dementia and 1 with motor neuron disease), 6 with progressive supranuclear palsy, 6 with spinocerebellar ataxia (1 associated with dementia), 4 with amyotrophic lateral sclerosis, 2 with Huntington's disease, 1 with MELAS, 1 with Lewy body dementia, and 1 with vascular dementia. The mean age at sample collection for this group was 63.8 ± 11.1 years (range 41-80). CSF samples were collected in polypropylene tubes following lumbar puncture at the L4/L5 or L3/L4 interspace with atraumatic needles after one night fasting. The samples were centrifuged at 3,000 g for 3 min at 4°C, aliquoted and stored at -80°C until analysis. CSF cell counts, glucose and protein concentration were determined. Albumin was measured by rate nephelometry. To evaluate the integrity of the blood brain barrier and the intrathecal IgG production, the albumin quotient (CSF albumin/serum albumin) X 10³ and the IgG index (CSF albumin/serum albumin)/(CSF IgG/serum IgG) were calculated. The study was conducted according to the provisions of the Helsinki Declaration and was approved by the Ethics Committee.

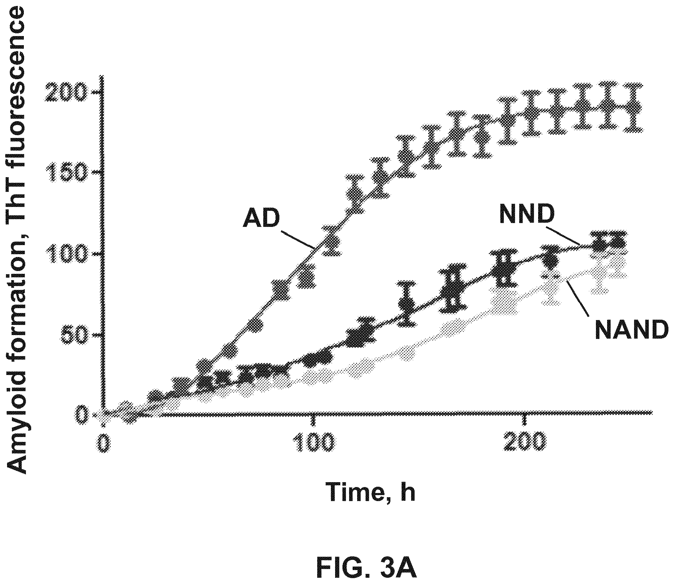

The experiments as well as the initial part of the analysis were conducted blind. **FIG. 3A** is a graph of amyloid formation versus time, measured as a function of ThT fluorescence labeling, showing the average kinetics of Aβ aggregation of 5 representative samples from the AD, NND, and NAND groups.

The results indicate that CSF from AD patients significantly accelerates Aβ aggregation as compared to control CSF (P<0.001). The significance of the differences in Aβ aggregation kinetics in the presence of human CSF samples was analyzed by one-way ANOVA, followed by the Tukey's multiple comparison post-test. The level of significance was set at P<0.05. The differences between AD and samples from the other two groups were highly significant with P<0.001 (***).

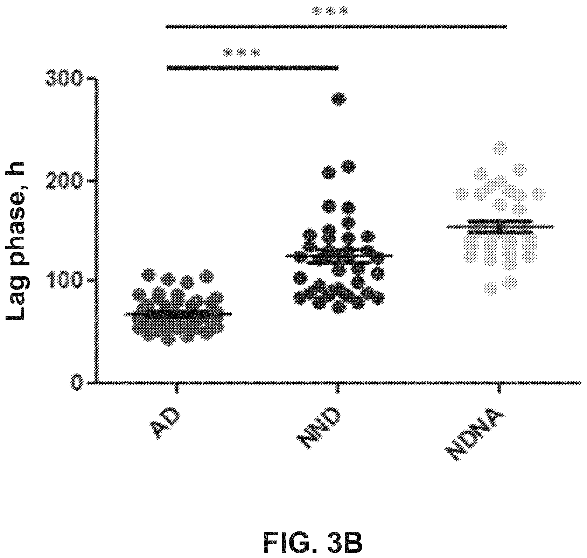

**FIG. 3B** is a graph of the lag phase time in h for samples from the AD, NND, and NAND groups. To determine the effect of individual samples on Aβ aggregation, the lag phase was estimated, defined as the time to ThT fluorescence larger than 40 arbitrary units after subtraction of a control buffer sample. This value was selected considering that it corresponds to ~5 times the reading of the control buffer sample.

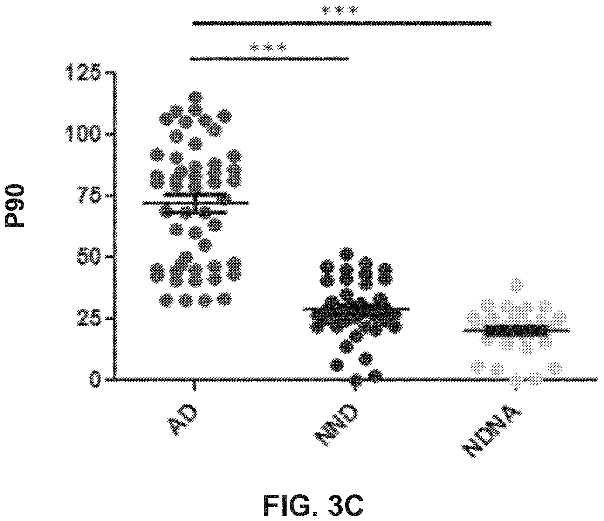

**FIG. 3C** is a graph showing the extent of amyloid formation obtained after 180 Aβ-PMCA cycles, i.e. 90 h of incubation (P90). Comparison of the lag phase and P90 among the experimental groups reveals a significant difference between AD and control samples from individuals with non-degenerative neurological diseases or with non-AD neurodegenerative diseases. Further, no correlation was detected between the aggregation parameters and the age of the AD patients, which indicates that the levels of the marker corresponds to aggregated Aβ protein in patient CSF, and not patient age.

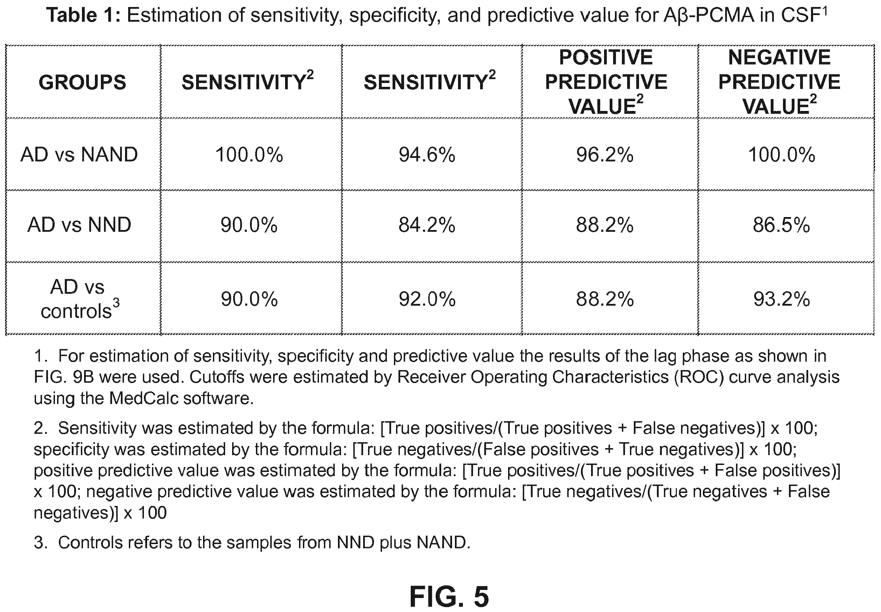

**FIG. 5****, Table** 1 shows estimations of the sensitivity, specificity and predictive value of the Aβ-PMCA test, calculated using the lag phase numbers.

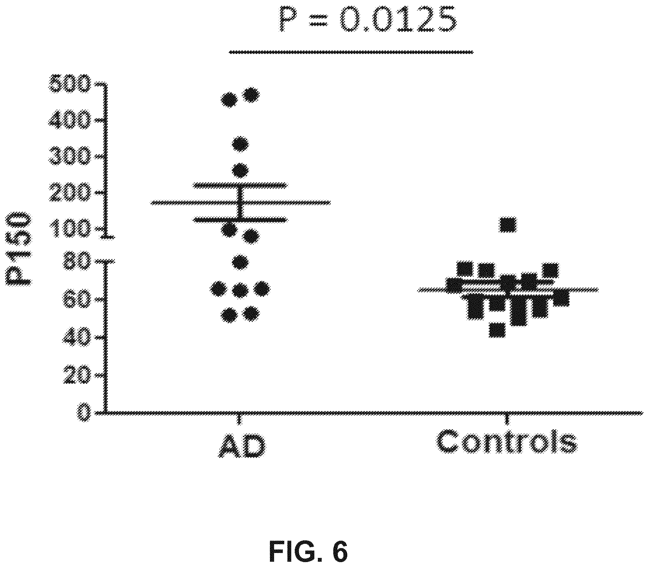

To study reproducibility, an experiment similar to the one shown in **FIGS. 3A-C** was independently done with different samples, reagents and a new batch of Aβ peptide as substrate for Aβ-PMCA. The extent of amyloid formation obtained after 300 Aβ-PMCA cycles, i.e. 150 h of incubation (P150), was measured in each patient. The control group includes both people affected by other neurodegenerative diseases and non-neurologically sick patients. Data for each sample represent the average of duplicate tubes. Statistical differences were analyzed by student-t test. **FIG. 6** is a graph of the lag phase time in h for samples obtained after 300 Aβ-PMCA cycles, i.e. 150 h of incubation (P90).

During the course of the study an entire set of CSF samples coming from a fourth location did not aggregate at all, even after spiking with large concentrations of synthetic oligomers. It is expected that reagent contamination during sample collection interfered with the assay.

The differences in aggregation kinetics between different samples were evaluated by the estimation of various different kinetic parameters, including the lag phase, A50, and P90. Lag phase is defined as the time required to reach a ThT fluorescence higher than 5 times the background value of the buffer alone. The A50 corresponds to the time to reach 50% of maximum aggregation. P90 corresponds to the extent of aggregation (measured as ThT fluorescence) at 90 h. Sensitivity, specificity and predictive value were determined using this data, with cutoff thresholds determined by Receiver Operating Characteristics (ROC) curve analysis, using MedCalc software (MedCalc Software, Ostend, Belgium).

### EXAMPLE 4: DETERMINATION OF THRESHOLD VALUES OF MISFOLDED Aβ FOR Aβ-PMCA DETECTION OF AD IN CSF

In support of **FIG. 5****, TABLE 1,** sensitivity, specificity and predictive value were determined using the lag phase data, with cutoff thresholds determined by Receiver Operating Characteristics (ROC) curve analysis, using the MedCalc software (version 12.2.1.0, MedCalc, Belgium). As shown in **FIG. 5****, TABLE 1,** a 90.0% sensitivity and 84.2% specificity was estimated for the control group consisting of age-matched individuals with non-degenerative neurological diseases. By contrast, for the clinically more relevant differentiation of AD from other neurodegenerative diseases including other forms of dementia, 100% sensitivity and 94.6% specificity was estimated. This ability of Aβ-PMCA to distinguish AD from other forms of neurodegenerative diseases is clinically significant. The overall sensitivity and specificity considering all control individuals was 90% and 92%, respectively.

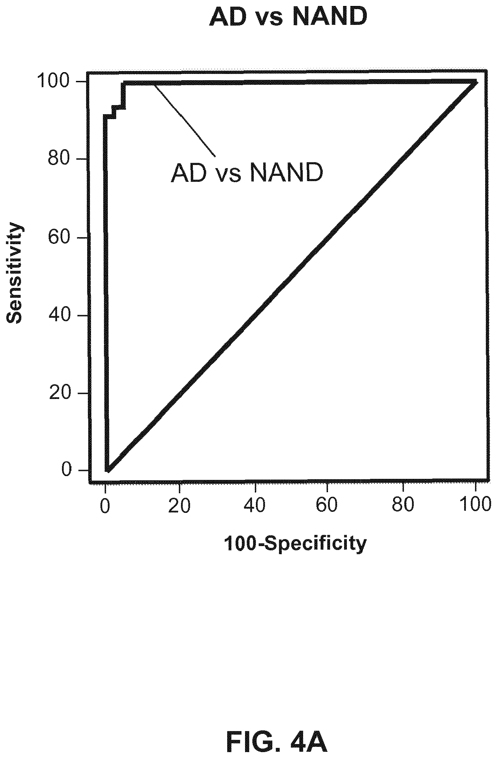

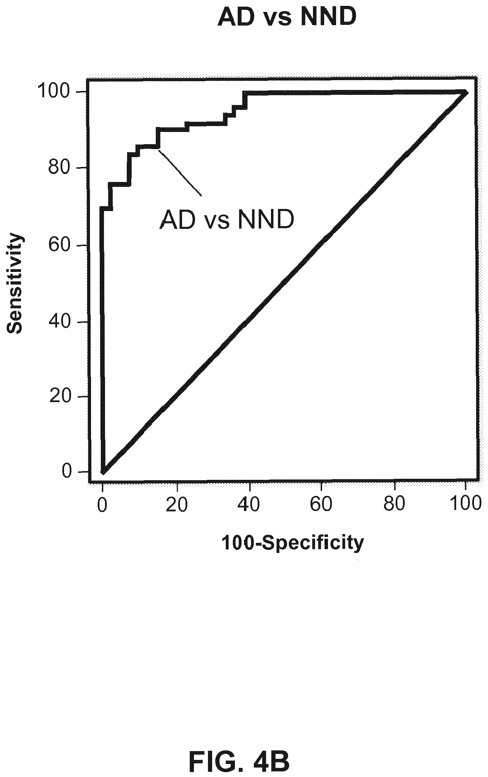

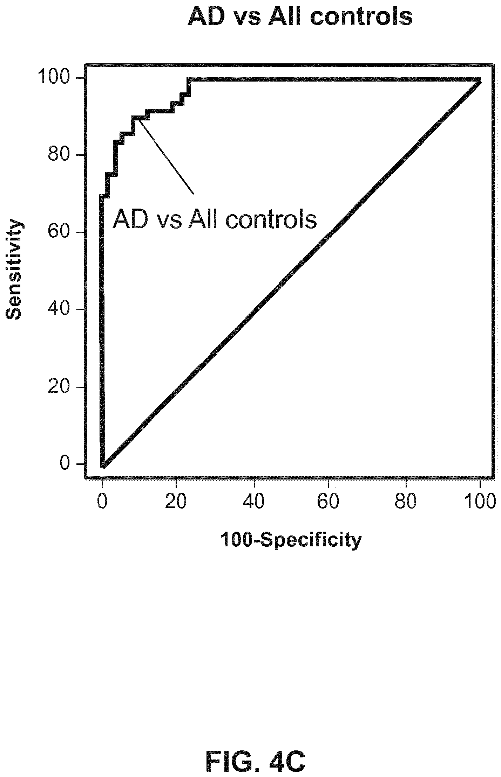

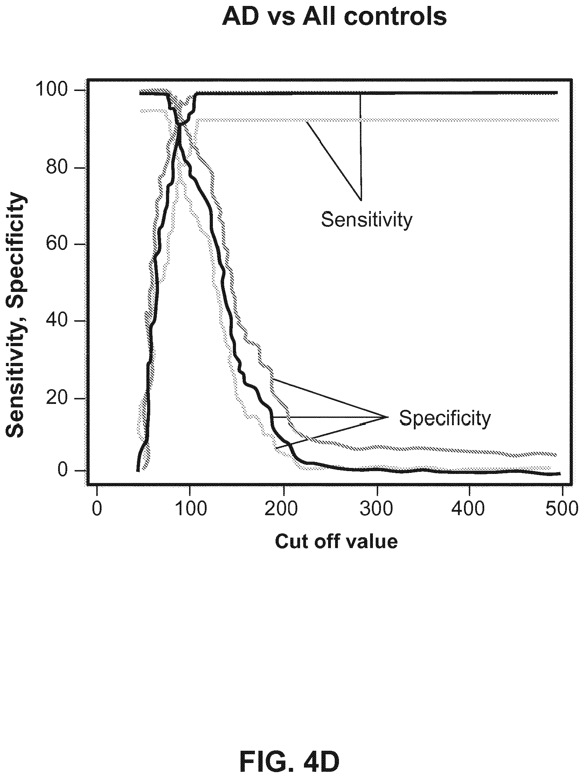

To evaluate the performance of the Aβ-PMCA test to distinguish AD patients from controls, the true positive rate (sensitivity) was plotted as a function of the false positive rate (specificity) for different cut-off points. For this analysis the lag phase values for AD vs NAND (**FIG. 4A**), AD vs NND (**FIG. 4B**) and AD vs All control samples (**FIG. 4C**) was used. The performance of the test, estimated as the area under the curve was 0.996 ± 0.0033, 0.95 ± 0.020 and 0.97 ± 0.011 for the comparison of AD with NAND, NND and all controls, respectively. Statistical analysis was done using the MedCalc ROC curve analysis software (version 12.2.1.0) and the result indicated that the test can distinguish AD from the controls with a P<0.0001. To estimate the most reliable cut-off point for the different set of group comparisons, sensitivity (blue line) and specificity (red line) were plotted for each cut-off value (**FIG. 4D**). The graph shows the curve and the 95% confidence intervals for the AD vs all control samples (including NAND and NND groups). These cut-off values were used to estimate sensitivity, specificity and predictive value in **FIG. 5****, Table 1**.

### EXAMPLE 5: Aβ-OLIGOMER IMMUNODEPLETION REMOVES Aβ SEEDS IN HUMAN CEREBROSPINAL FLUID AND CONFIRMS Aβ-PMCA DETECTS SOLUBLE MISFOLDED Aβ PROTEIN IN AD CSF

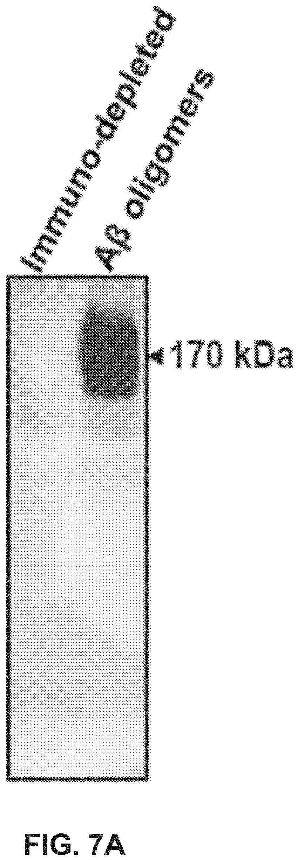

Immunodepletion experiments were performed to confirm that Aβ-PMCA detects a seeding activity associated to soluble, misfolded Aβ protein present in CSF. The methodology for efficient immunodepletion of soluble, misfolded Aβ protein was first optimized by using synthetically prepared soluble, misfolded Aβ protein. Immunodepletion was performed by incubation with dynabeads conjugated with a mixture of antibodies recognizing specifically the sequence of Aβ (4G8) and conformational (A11) antibodies. **FIG. 7A** is a western blot showing results of immunodepletion using synthetically prepared Aβ oligomers spiked into human CSF. Soluble, misfolded Aβ protein was efficiently removed by this immunodepletion.

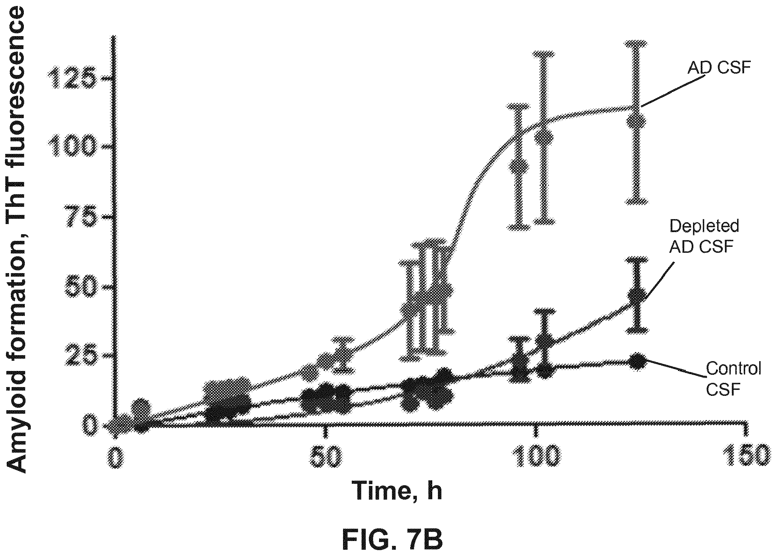

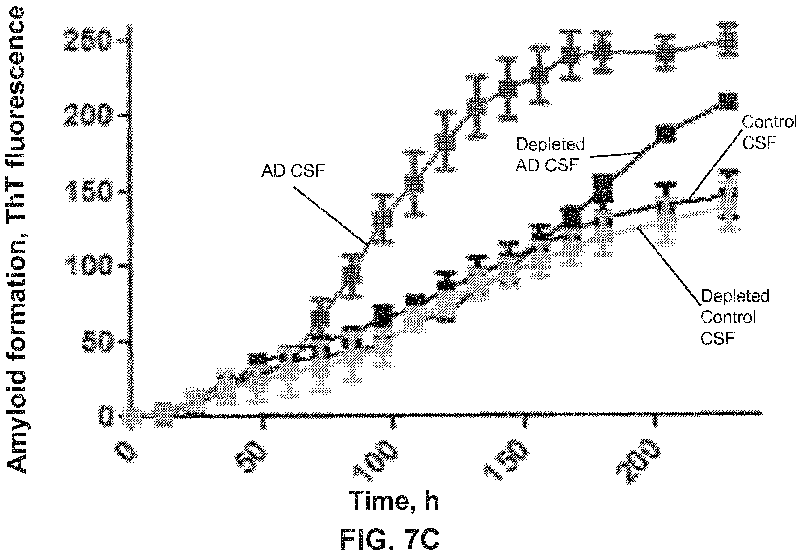

**FIGs. 7A** and **7B** are graphs of amyloid formation versus time as measured by Thioflavin T fluorescence, demonstrating that seeding activity in AD CSF is removed by soluble, misfolded Aβ protein immuno-depletion. Samples of AD CSF before or after immunodepletion with 4G8 and A11 antibodies were used to seed Aβ aggregation in the Aβ-PMCA assay. Immunodepletion was applied to 3 AD CSF. **FIG. 7B** is a graph showing the kinetics of control and immunodepleted CSF samples. **FIG. 7B** shows that for immunodepleted AD CSF, the kinetics of Aβ aggregation in the AP-PMCA reaction was comparable to that observed in control CSF samples, and both were significantly different from the aggregation observed with AD CSF prior to immunodepletion. **FIG. 7C** is a graph showing the kinetics of control and immunodepleted CSF samples, depleted only with the A11 conformational antibody and aggregation monitored by Aβ-PMCA assay. **FIG. 7C** shows similar results, obtained using AD CSF immunodepleted using the A11 conformational antibody, which specifically recognizes, misfolded Aβ. These results confirm that Aβ-PMCA detects soluble, misfolded β protein in AD CSF.

### EXAMPLE 6: SOLID PHASE IMMUNO CAPTURING

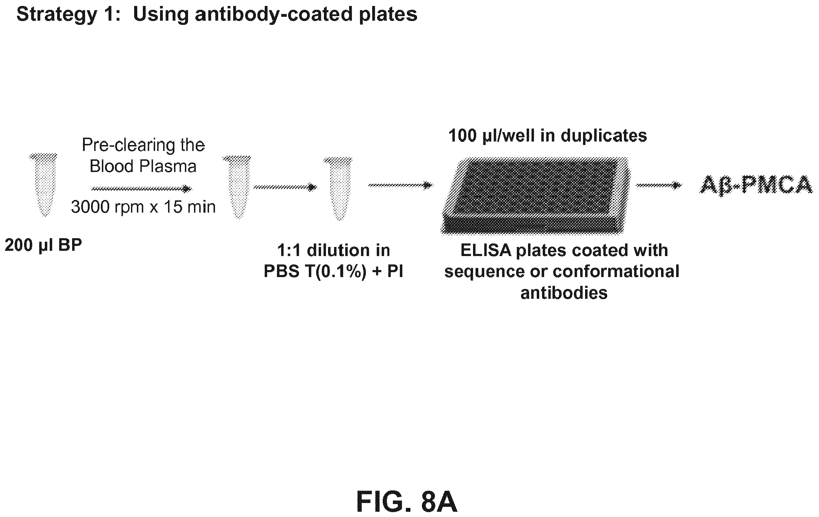

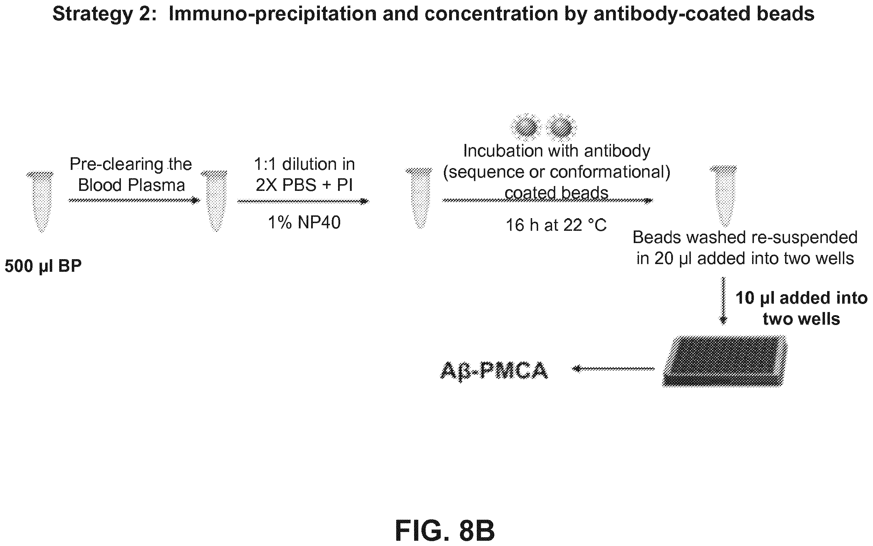

**FIGs. 8A** and **8B** are schematic representations of two solid phase methods used to capture soluble, misfolded Aβ protein from complex samples such as blood plasma. Strategy 1 employed ELISA plates pre-coated with specific antibodies bound to a solid phase on the ELISA plate. After washing the plates, the Aβ-PMCA reaction was carried out in the same plates. Strategy 2 used magnetic beads as the solid phase coated with specific antibodies. This approach provided concentration of the samples.

### EXAMPLE 7: SPECIFICITY OF IMMUNO CAPTURING

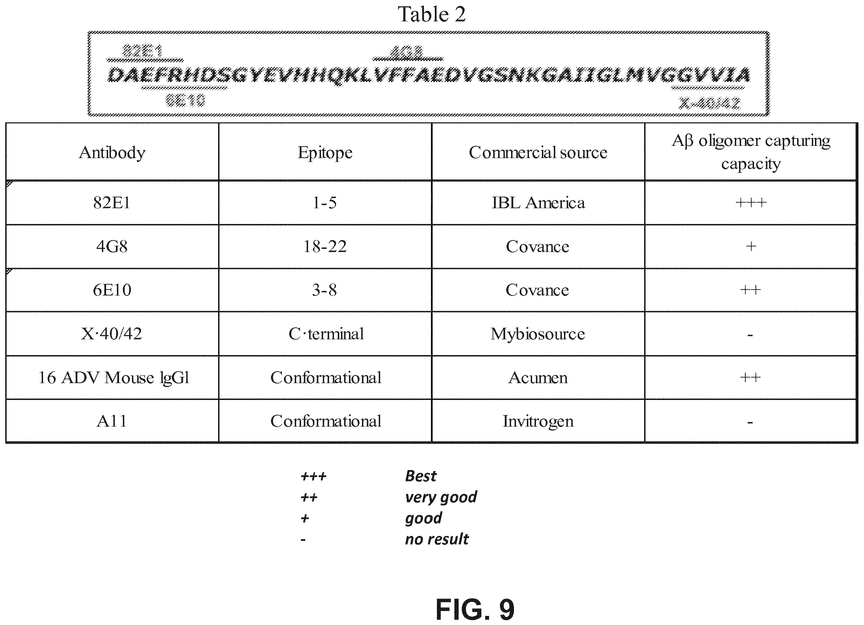

**FIG. 9** shows **Table 2,** demonstrating the ability of specific antibodies to capture the Aβ oligomers. The top panel shows a schematic representation of the epitope recognition site on the Aβ protein of the diverse sequence antibodies used in this study. **Table 2** in **FIG. 9** demonstrates the efficiency of different sequence or conformational antibodies to capture Aβ oligomers. The capacity to capture oligomers was measured by spiking synthetic Aβ oligomers in healthy human blood plasma and detection by Aβ-PMCA. The symbols indicate that the detection limits using the different antibodies were: <12 fmol (+++); between 10-100 fmol (++); >1pmol (+) and not significantly higher than without capturing reagent (-).

### EXAMPLE 8: DETECTION OF Aβ OLIGOMERS SPIKED IN HUMAN PLASMA

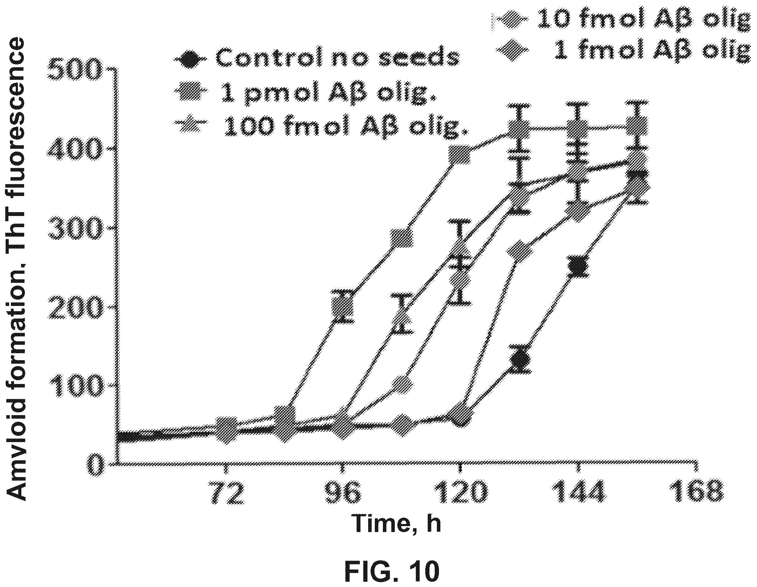

**FIG. 10** is a graph of amyloid formation versus time as measured by Thioflavin T fluorescence showing detection of soluble, misfolded Aβ protein spiked in human plasma. ELISA plates pre-coated with protein G were coated with an anti-conformational antibody (16ADV from Acumen). Thereafter, plates were incubated with human blood plasma (100 µl) as such (control) or spiked with different concentrations of synthetic soluble, misfolded Aβ protein. After incubation, plates were subjected to Aβ-PMCA (29 min incubation and 30 s shaking) in the presence of Aβ40 monomer (2 µM) and ThT (5 µM). Amyloid formation was measured by Thioflavin fluorescence. **FIG. 10** is representative of several experiments done with 3 different antibodies which worked similarly.

### EXAMPLE 9: CAPTURING OF SOLUBLE MISFOLDED Aβ FROM AD PATIENT SAMPLES VS CONTROLS

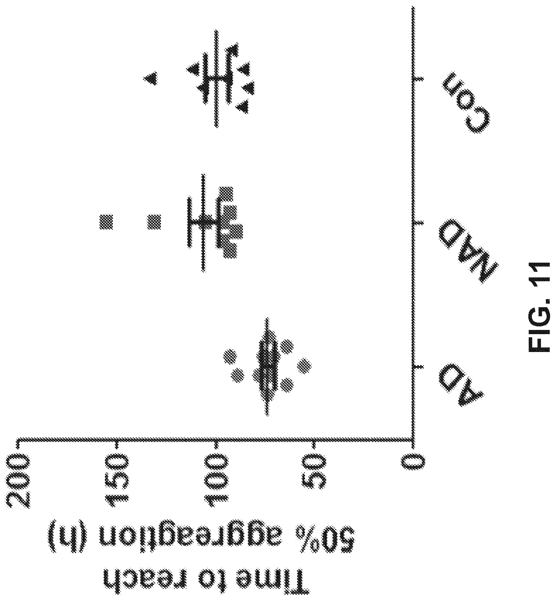

**FIG. 11** is a graph showing time to reach 50% aggregation in an Aβ-PMCA assay in plasma samples from AD patients and controls. Blood plasma samples from patients affected by AD, non-AD neurodegenerative diseases (NAD), and healthy controls were incubated with anti-Aβ antibody (82E1) coated beads. Aβ-PMCA was carried out as described in **EXAMPLE 2.** The time needed to reach 50% aggregation was recorded in individual patients in each group. Differences were analyzed by one-way ANOVA followed by the Tukey's post-hoc test. ROC analysis of this data showed a 82% sensitivity and 100% specificity for correctly identifying AD patients from controls.

### EXAMPLE 10: SONICATION AND SHAKING ARE EFFECTIVE WITH VARIOUS DETECTION METHODS

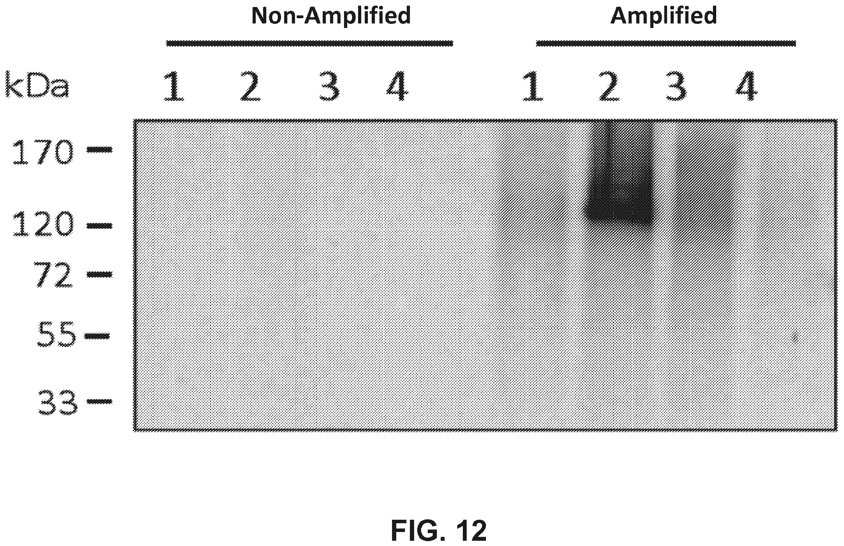

**FIG. 12** is a western blot showing the results of amplification of Aβ aggregation by using sonication instead of shaking as a mean to fragment aggregates. The experiment was done in the presence of distinct quantities of synthetic Aβ oligomers. Samples of 10 µg/ml of seed-free monomeric Aβ1-42 were incubated alone (lane 1) or with 300 (lane 2), 30 (lane 3) and 3 (lane 4) fmols of, misfolded Aβ. Samples were either frozen without amplification (non-amplified) or subjected to 96 PMCA cycles (amplified), each including 30 min incubation followed by 20 sec sonication. Aggregated Aβ was detected by western blot using anti-Aβ antibody after treatment of the samples with proteinase K (PK). In our experiments, it was observed that detection using thioflavin T fluorescence was not compatible with sonication, but works very well with shaking as a physical disruption method. **FIG. 12** shows that using a different detection method for the Aβ aggregates, in this case Western Blotting, sonication works as well as shaking.

### EXAMPLE 11: PRODUCTION OF MONOMERIC Aβ AS PMCA SUBSTRATE

Seed-free monomeric Aβ was obtained by size exclusion chromatography. Briefly, an aliquot of a 1 mg/mL peptide solution prepared in dimethylsulfoxide was fractionated using a Superdex 75 column eluted at 0.5 mL/min with 10 mM sodium phosphate at pH 7.5. Peaks will be detected by UV absorbance at 220 nm. The peak corresponding to 4-10 kDa molecular weight containing monomer/dimmers of Aβ was collected and concentration determined by amino acid analysis. Samples were stored lyophilized at -80 °C.

### EXAMPLE 12: PRODUCTION AND PURIFICATION OF Aβ

E. coli cells harboring pET28 GroES-Ub-Aβ42 plasmid were grown in Luria broth (LB) at 37 °C, and expression was induced with 0.4 mM IPTG. After 4 h, cells were harvested and lysed in a lysis buffer (20 mM Tris-Cl, pH 8.0, 10 mM NaCl, 0.1 mM PMSF, 0.1 mM EDTA and 1 mM β-mercaptoethanol) and centrifuged at 18,000 rpm for 30 min. Inclusion bodies were re-suspended in a resuspension buffer (50 mM Tris-Cl, pH 8.0, 150 mM NaCl, and 1 mM DTT) containing 6 M urea. Insoluble protein was removed by centrifugation at 18,000 rpm for 30 min. The supernatant containing GroES-Ub-Aβ42 fusion protein will be collected. To cleave off Aβ42 from fusion protein, the fusion protein was diluted 2-fold with resuspension buffer and treated with recombinant de-ubiquinating enzyme (Usp2cc) 1: 100 enzyme to substrate molar ratio at 37 °C for 2 h. After that, samples was loaded on a C18 column (25 mm x 250 mm, Grace Vydac, USA). Aβ42 was purified with a solvent system buffer 1 (30 mM ammonium acetate, pH 10, 2% acetonitrile) and buffer 2 (70% acetonitrile) at a flow rate 10 ml/min using a 20-40% linear gradient of buffer 2 over 35 min. Purified Aβ42 was lyophilized and stored at -80 °C, until use.

### EXAMPLE 13: DETECTION OF αS SEEDS BY PD-PMCA

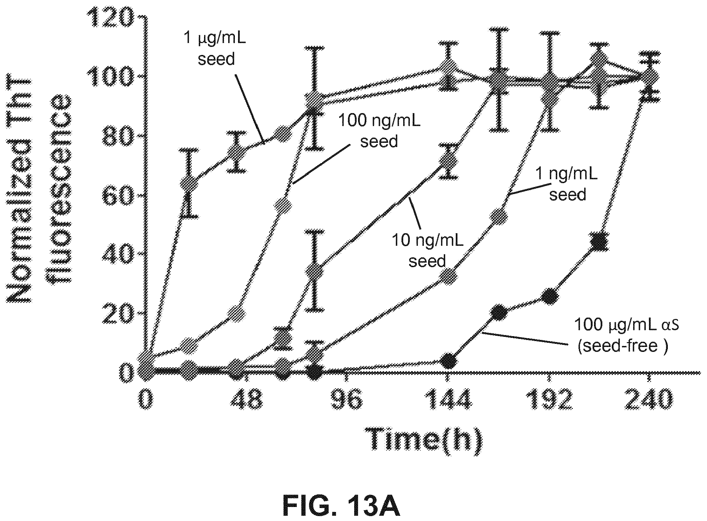

**EXAMPLE 13A:** Seeding of αS aggregation was studied by incubating a solution of seed-free αS in the presence of Thioflavin T with or without different quantities of synthetic soluble oligomeric αS protein: Control (no αS oligomer); or 1 ng/mL, 10 ng/mL, 100 ng/mL, and 1 µg/mL of the synthetic soluble oligomeric αS protein seed. αS-PMCA general procedure: Solutions of 100 µg/mL αS seed-free αS in PBS, pH 7.4 (200 µL total volume) were placed in opaque 96-wells plates and incubated alone or in the presence of the indicated concentrations of synthetic αS aggregates or 40 µL of CSF aliquots. Samples were incubated in the presence of 5 µM Thioflavin T (ThT) and subjected to cyclic agitation (1 min at 500 rpm followed by 29 min without shaking) using an Eppendorf thermomixer, at a constant temperature of 22 °C. At various time points, ThT fluorescence was measured in the plates at 485 nm after excitation at 435 nm using a plate spectrofluorometer. **FIG. 13A** is a graph of Thioflavin T fluorescence as a function of time, showing the detection of αS seeds by PD-PMCA, using the indicated concentration of synthetic soluble oligomeric αS protein seeds. The peptide concentration, temperature and pH of the buffer were monitored to control the extent of the lag phase and reproducibility among experiments. Aggregation of monomeric αS protein was observed in the presence of 1 ng/mL, 10 ng/mL, 100 ng/mL, and 1 µg/mL αS of the synthetic soluble oligomeric αS protein seed.

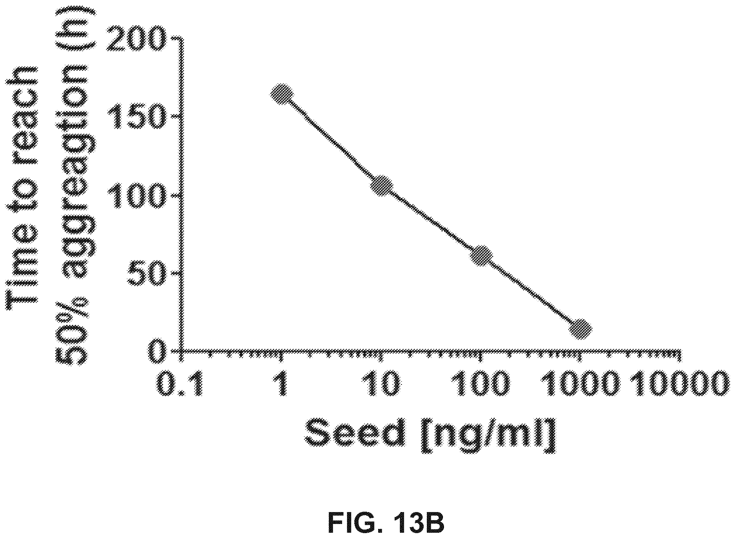

**EXAMPLE 13B:** The time to reach 50% aggregation as a function of amounts of αS seeds added was determined using the samples in **EXAMPLE 1A.** **FIG. 13B** is a graph showing time to reach 50% aggregation plotted as a function of amounts of αS seeds added.

### EXAMPLE 14: αS-PMCA DETECTS OLIGOMERIC αS IN THE CEREBROSPINAL FLUID OF PD PATIENTS

Detection of seeding activity in human CSF samples from controls and PD patients was performed by PD-PMCA. Purified seed free alpha-synuclein (100 µg/mL) in PBS, pH 7.4 was allowed to aggregate at 37 °C with shaking at 500 rpm in the presence of CSF from human patients with confirmed PD, AD or non-neurodegenerative neurological diseases (NND). The extend of aggregation was monitored by Thioflavin fluorescence at 485 nm after excitation at 435 nm using a plate spectrofluorometer.

Aliquots of CSF were obtained from PD patients, cognitively normal individuals affected by non-degenerative neurological diseases (NND), and patients affected by Alzheimer's disease (AD). Test CSF samples were obtained from patients with the diagnosis of probable PD as defined by the DSM-IV and determined using a variety of tests, including routine medical examination, neurological evaluation, neuropsychological assessment, and magnetic resonance imaging. CSF samples were collected in polypropylene tubes following lumbar puncture at the L4/L5 or L3/L4 interspace with atraumatic needles after one night fasting. The samples were centrifuged at 3,000 g for 3 min at 4°C, aliquoted and stored at -80°C until analysis. CSF cell counts, glucose and protein concentration were determined. Albumin was measured by rate nephelometry. To evaluate the integrity of the blood brain barrier and the intrathecal IgG production, the albumin quotient (CSF albumin/serum albumin) X 10³ and the IgG index (CSF albumin/serum albumin)/(CSF IgG/serum IgG) were calculated. The study was conducted according to the provisions of the Helsinki Declaration and was approved by the Ethics Committee.

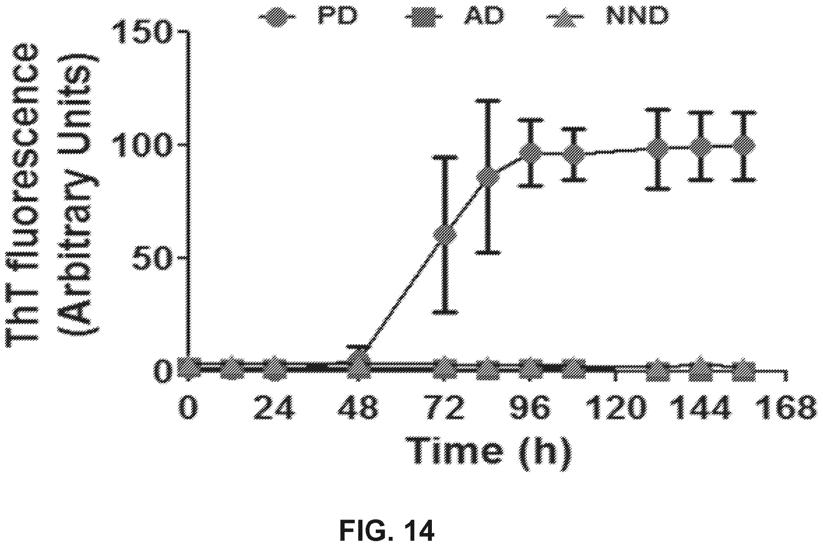

The experiments as well as the initial part of the analysis were conducted blind. **FIG. 14** is a graph of αS oligomerization versus time, measured as a function of ThT fluorescence labeling, showing the average kinetics of αS aggregation of representative samples from the PD, AD, and NND groups.

The results indicate that CSF from PD patients significantly accelerates αS aggregation as compared to control CSF (P<0.001). The significance of the differences in αS aggregation kinetics in the presence of human CSF samples was analyzed by one-way ANOVA, followed by the Tukey's multiple comparison post-test. The level of significance was set at P<0.05. The differences between PD and samples from the other two groups were highly significant with P<0.001 (***).

### EXAMPLE 15: SPECIFICITY OF IMMUNO CAPTURING

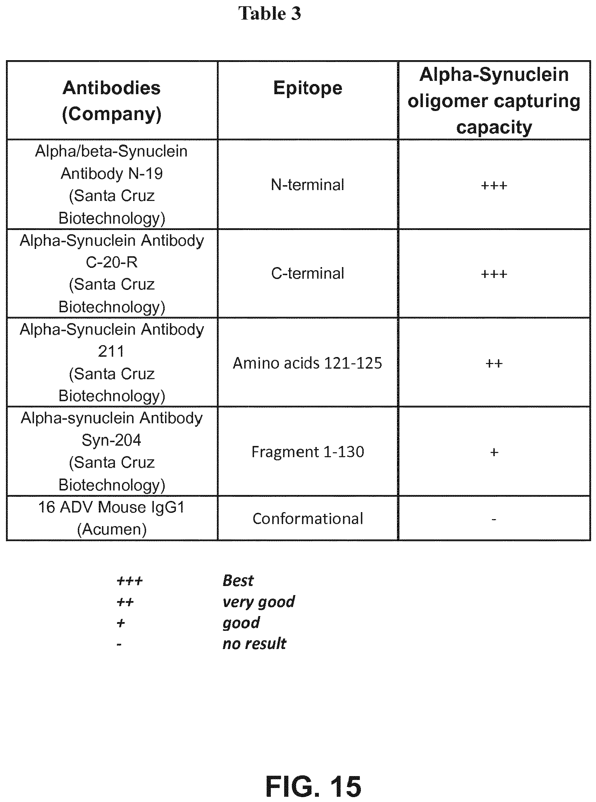

**FIG. 15** shows **Table 3,** demonstrating the ability of different sequence or conformational antibodies to capture αS oligomers. The capacity to capture oligomers was measured by spiking synthetic αS oligomers in healthy human blood plasma and detection by αS-PMCA. The first column shows various antibodies tested and corresponding commercial sources. The second column lists the epitope recognition site on the αS protein of the diverse sequence antibodies used in this study. The third column indicates the observed ability of specific antibodies to capture the αS oligomers. The symbols indicate that the detection limits using the different antibodies were: <12 fmol (+++); between 10-100 fmol (++); > 1 pmol (+) and not significantly higher than without capturing reagent (-). Alpha/beta-synuclein antibody N-19 (N-terminal epitope) and alpha-synuclein antibody C-20-R (C-terminal epitope) showed the best results; and alpha-synuclein antibody 211 (epitope: amino acids 121-125) showed very good results; alpha-synuclein antibody 204 (epitope: fragment 1-130) showed good results; and 16 ADV Mouse IgG1 (conformational epitope) showed no result.

### EXAMPLE 16: SOLID PHASE IMMUNO CAPTURING

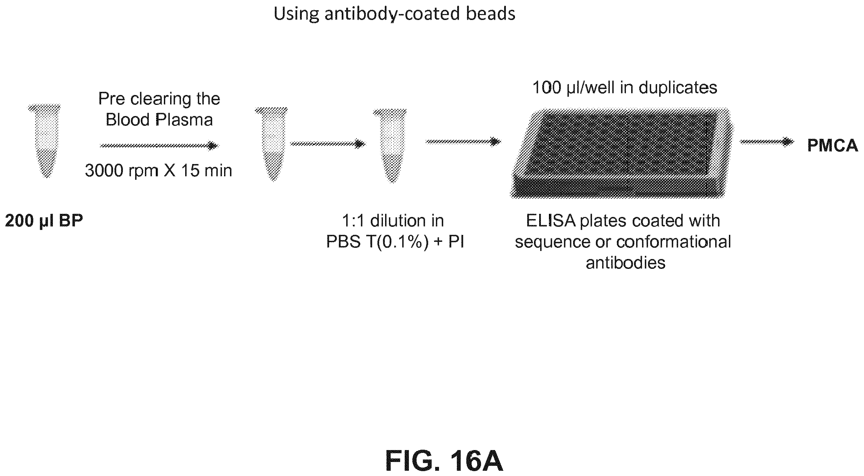

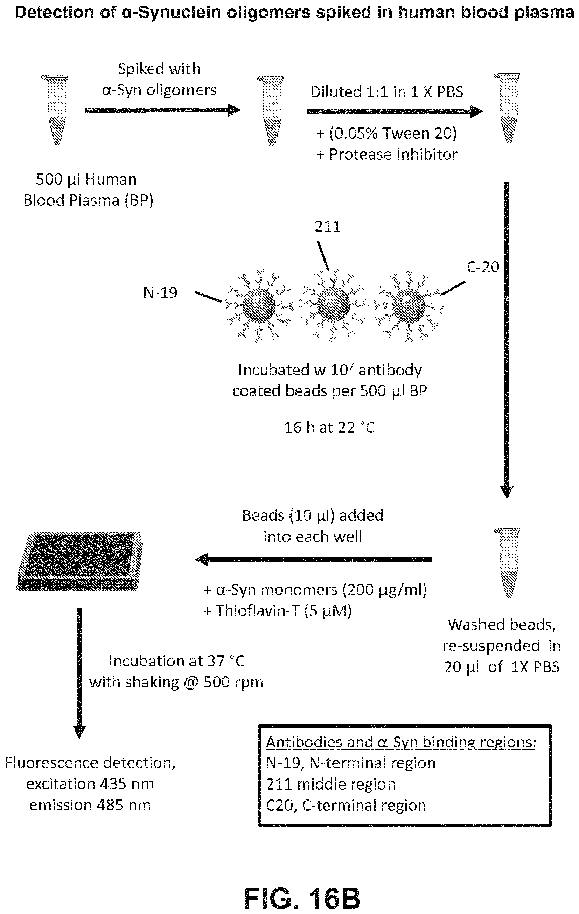

**FIGs. 16A** and **16B** are schematic representations of two solid phase methods used to capture soluble, misfolded αS protein from complex samples such as blood plasma. Strategy 1 employed ELISA plates pre-coated with specific antibodies bound to a solid phase on the ELISA plate. After washing the plates, the αS-PMCA reaction was carried out in the same plates. Strategy 2 used magnetic beads as the solid phase coated with specific antibodies. This approach provided concentration of the samples.

### EXAMPLE 17: αS-PMCA FOR THE DETECTION OF α-SYNUCLEIN OLIGOMERS SPIKED IN HUMAN BLOOD PLASMA

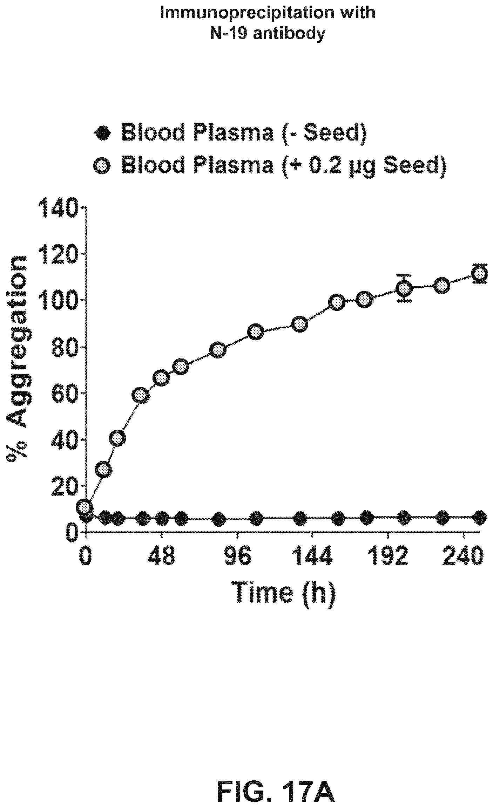

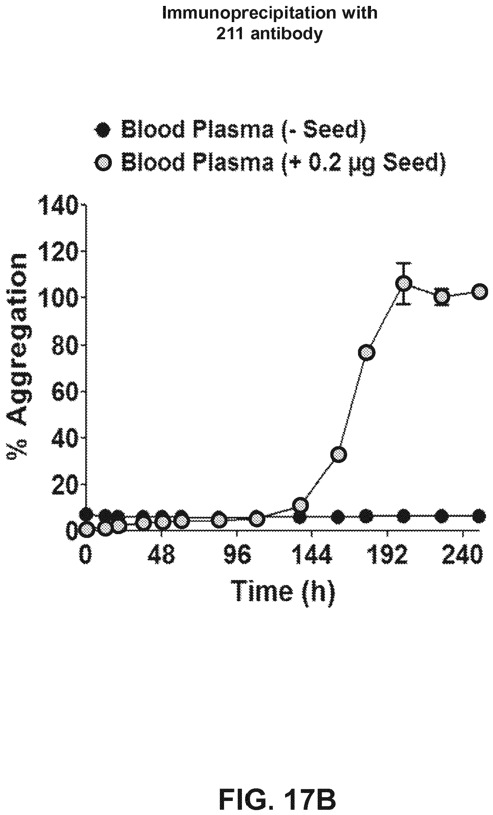

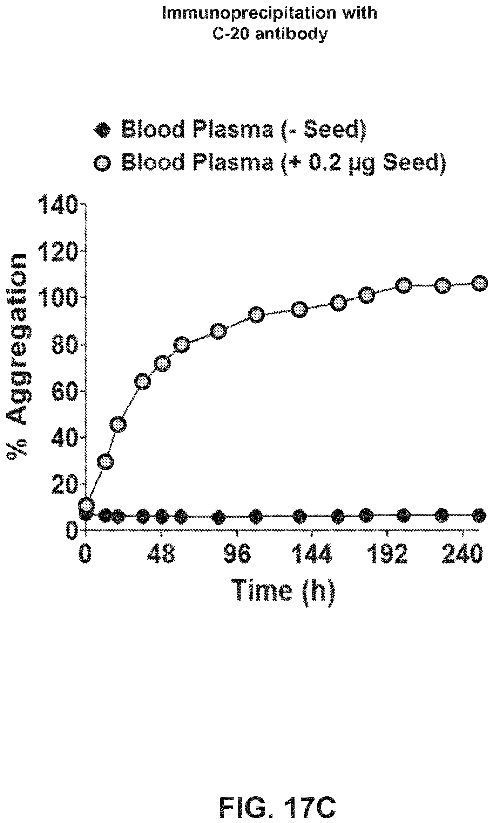

Immunoprecipitation of α-Synuclein oligomers from human blood plasma was performed by anti-α-Synuclein antibody-coated beads (Dynabeads) and a seeding aggregation assay using α-Synuclein monomers as seeding substrate along with thioflavin-T for detection. The anti-α-Synuclein coated beads (1x10' beads) were incubated with human blood plasma (500 µL) with α-Synuclein seeds (+ 0.2 µg Seed) and without α-Synuclein seeds (- Seed). After immunoprecipitation, the beads were re-suspended in 20 µL of reaction buffer (1X PBS), and 10 µL of beads were added to each well of a 96-well plate. The aggregation assay was performed by adding α-Synuclein monomers (200 µg/mL) and thioflavin-T (5 µM). The increase in florescence was monitored by a fluorimeter using an excitation of 435 nm and emission of 485 nm. **FIG. 17A** illustrates immunoprecipitation/aggregation results with N-19 antibody in blood plasmas with and without seed. **FIG. 17B** illustrates immunoprecipitation/aggregation results with 211 antibody in blood plasmas with and without seed. **FIG. 17C** illustrates immunoprecipitation/aggregation results with C-20 antibody in blood plasmas with and without seed.

### EXAMPLE 18: αS-PMCA DETECTS OLIGOMERIC αS IN THE CEREBROSPINAL FLUID OF PATIENTS AFFECTED BY PD AND MULTIPLE SYSTEM ATROPHY WITH HIGH SENSITIVITY AND SPECIFICITY.

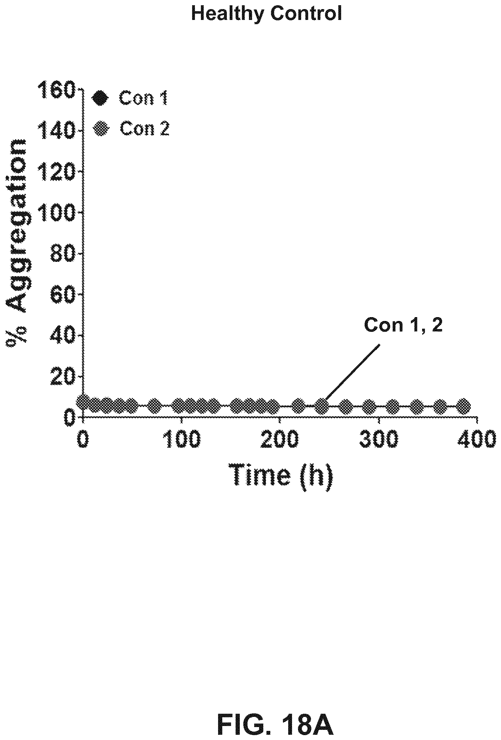

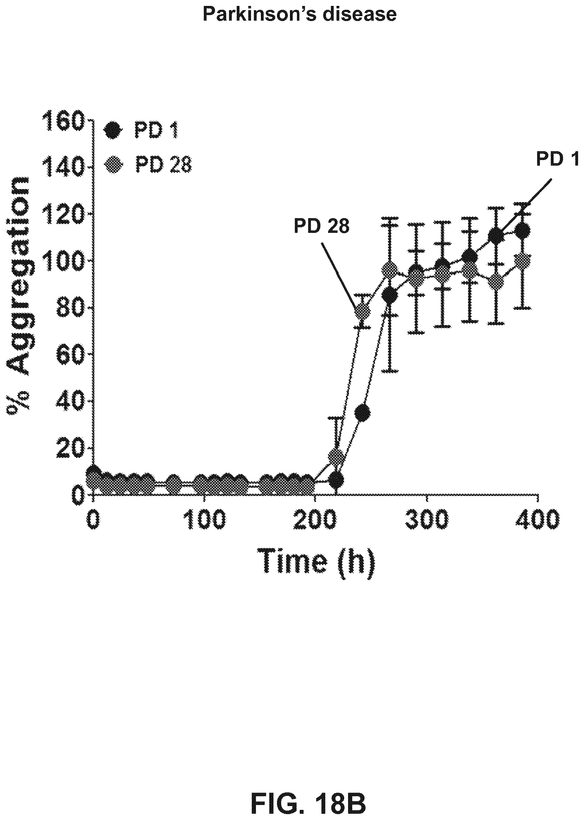

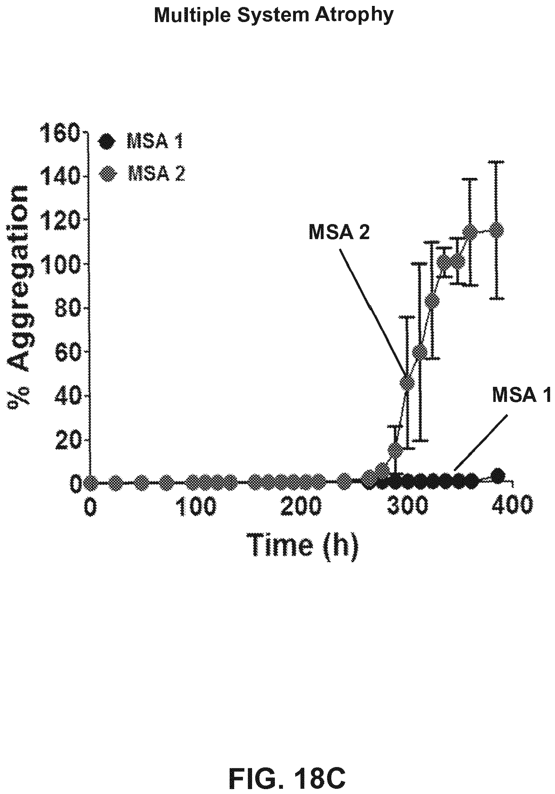

To study the efficiency of αS-PMCA for biochemical diagnosis of PD and related α-synucleinopathies, such as multiple system atrophy (MSA), tests were performed on CSF from many patients affected by these diseases as well as controls affected by other diseases. **FIGS. 18A****,** **18B****,** and **18C** show detection of seeding activity in human CSF samples from controls and patients affected by PD and MSA, respectively, using αS-PMCA. Purified seed free alpha-synuclein (100 µg/mL) in buffer MES, pH 6.0 was allowed to aggregate at 37 °C with shaking at 500 rpm in the presence of CSF from human patients and controls. The extent of aggregation was monitored by Thioflavin T fluorescence at 485 nm after excitation at 435 nm using a plate spectrofluorometer.

Test CSF samples were obtained from patients with the diagnosis of probable PD and MSA as defined by the DSM-IV and determined using a variety of tests, including routine medical examination, neurological evaluation, neuropsychological assessment, and magnetic resonance imaging. CSF samples were collected in polypropylene tubes following lumbar puncture at the L4/L5 or L3/L4 interspace with atraumatic needles after one night fasting. The samples were centrifuged at 3,000 g for 3 min at 4°C, aliquoted and stored at - 80°C until analysis. CSF cell counts, glucose and protein concentration were determined. Albumin was measured by rate nephelometry. The study was conducted according to the provisions of the Helsinki Declaration and was approved by the Ethics Committee.

The experiments as well as the initial part of the analysis were conducted blind. **FIGS. 18A****,** **18B****,** and **18C** are graphs of αS aggregation versus time, measured as a function of ThT fluorescence labeling, showing the average kinetics of αS aggregation, respectively, for controls and two representative samples from the PD and MSA groups.

The results indicate that CSF from PD patients significantly accelerates αS aggregation as compared to control CSF (P<0.001). The significance of the differences in αS aggregation kinetics in the presence of human CSF samples was analyzed by one-way ANOVA, followed by the Tukey's multiple comparison post-test. The level of significance was set at P<0.05. The differences between PD and samples from the other two groups were highly significant with P<0.001 (***).

The outcome of the overall set of 29 PD or MSA samples and 41 controls was that 26 of the 29 PD or MSA samples were positive, whereas 3 of the 41 control samples were positive, which corresponded to a 90% sensitivity and 93% specificity.

To the extent that the term "includes" or "including" is used in the specification or the claims, it is intended to be inclusive in a manner similar to the term "comprising" as that term is interpreted when employed as a transitional word in a claim. Furthermore, to the extent that the term "or" is employed (e.g., A or B) it is intended to mean "A or B or both." When the applicants intend to indicate "only A or B but not both" then the term "only A or B but not both" will be employed. Thus, use of the term "or" herein is the inclusive, and not the exclusive use. See Bryan A. Garner, A Dictionary of Modern Legal Usage 624 (2d. Ed. 1995). Also, to the extent that the terms "in" or "into" are used in the specification or the claims, it is intended to additionally mean "on" or "onto." To the extent that the term "selectively" is used in the specification or the claims, it is intended to refer to a condition of a component wherein a user of the apparatus may activate or deactivate the feature or function of the component as is necessary or desired in use of the apparatus. To the extent that the term "operatively connected" is used in the specification or the claims, it is intended to mean that the identified components are connected in a way to perform a designated function. To the extent that the term "substantially" is used in the specification or the claims, it is intended to mean that the identified components have the relation or qualities indicated with degree of error as would be acceptable in the subject industry.

As used in the specification and the claims, the singular forms "a," "an," and "the" include the plural unless the singular is expressly specified. For example, reference to "a compound" may include a mixture of two or more compounds, as well as a single compound.

As used herein, the term "about" in conjunction with a number is intended to include ± 10% of the number. In other words, "about 10" may mean from 9 to 11.

As used herein, the terms "optional" and "optionally" mean that the subsequently described circumstance may or may not occur, so that the description includes instances where the circumstance occurs and instances where it does not.

In addition, where features or aspects of the disclosure are described in terms of Markush groups, those skilled in the art will recognize that the disclosure is also thereby described in terms of any individual member or subgroup of members of the Markush group. As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, and the like. As a non-limiting example, each range discussed herein can be readily broken down into a lower third, middle third and upper third, and the like. As will also be understood by one skilled in the art all language such as "up to," "at least," "greater than," "less than," include the number recited and refer to ranges which can be subsequently broken down into sub-ranges as discussed above. Finally, as will be understood by one skilled in the art, a range includes each individual member. For example, a group having 1-3 cells refers to groups having 1, 2, or 3 cells. Similarly, a group having 1-5 cells refers to groups having 1, 2, 3, 4, or 5 cells, and so forth. While various aspects and embodiments have been disclosed herein, other aspects and embodiments will be apparent to those skilled in the art.

As stated above, while the present application has been illustrated by the description of embodiments thereof, and while the embodiments have been described in considerable detail, it is not the intention of the applicants to restrict or in any way limit the scope of the appended claims to such detail. Additional advantages and modifications will readily appear to those skilled in the art, having the benefit of the present application. Therefore, the application, in its broader aspects, is not limited to the specific details, illustrative examples shown, or any apparatus referred to. Departures may be made from such details, examples, and apparatuses without departing from the spirit or scope of the general inventive concept.

The various aspects and embodiments disclosed herein are for purposes of illustration and are not intended to be limiting, with the true scope and spirit being indicated by the following claims.

The invention may be defined by one of the following aspects:
1. A method for determining a presence of a soluble, misfolded protein in a sample, comprising:
   contacting the sample with a monomeric, folded protein corresponding to the soluble, misfolded protein to form an incubation mixture;
   conducting an incubation cycle two or more times effective to form an amplified portion of misfolded protein, each incubation cycle comprising:
      incubating the incubation mixture effective to cause misfolding and/or aggregation of at least a portion of the monomeric, folded protein in the presence of the soluble, misfolded protein;
      physically disrupting the incubation mixture effective to break up at least a portion of any protein aggregate present; and
      determining the presence of the soluble, misfolded protein in the sample by detecting at least a portion of the soluble, misfolded protein,
      the soluble, misfolded protein comprising one or more of: a soluble, misfolded monomer and a soluble, misfolded aggregate; and
      the amplified portion of misfolded protein comprising one or more of: an amplified portion of the soluble, misfolded monomer, an amplified portion of the soluble, misfolded aggregate, and an insoluble, misfolded aggregate,
      provided that the monomeric, folded protein and the soluble, misfolded protein exclude prion protein (PrP) and isoforms thereof.
2. The method of aspect 1:
   further comprising contacting an indicator of the soluble, misfolded protein to the incubation mixture, the indicator of the soluble, misfolded protein being characterized by an indicating state in the presence of the soluble, misfolded protein and a non-indicating state in the absence of the soluble, misfolded protein; and
   wherein the determining the presence of the soluble, misfolded protein in the sample comprises detecting the indicating state of the indicator of the soluble, misfolded protein.
3. The method of aspect 2, the indicating state of the indicator and the non-indicating state of the indicator being characterized by a difference in fluorescence, the determining the presence of the soluble, misfolded protein in the sample comprising detecting the difference in fluorescence.
4. The method of aspect 2, comprising contacting a molar excess of the indicator of the soluble, misfolded protein to the incubation mixture, the molar excess being greater than a total molar amount of protein monomer included in the monomeric, folded protein and the soluble, misfolded protein in the incubation mixture.
5. The method of aspect 2, the indicator of the soluble, misfolded protein comprising one or more of: Thioflavin T, Congo Red, m-I-Stilbene, Chrysamine G, PIB, BF-227, X-34, TZDM, FDDNP, MeO-X-04, IMPY, NIAD-4, luminescent conjugated polythiophenes, a fluorescent protein, and derivatives thereof.
6. The method of aspect 1, the determining the presence of the soluble, misfolded protein in the sample comprising determining an amount of the soluble, misfolded protein in the sample.
7. The method of aspect 6, comprising detecting the amount of the soluble, misfolded protein in the sample at a sensitivity of at least about one or more of: 80%, 81%, 82%, 83%, 84%, 85%, 86%, 87%, 88%, 89%, 90%, 91%, 92%, 93%, 94%, 95%, 96%, 97%, 98%, 99%, and 100%.
8. The method of aspect 6, comprising detecting the amount of the soluble, misfolded protein in the sample at less than about one or more of: 100 nmol, 10 nmol, 1 nmol, 100 pmol, 10 pmol, 1 pmol, 100 fmol, 10 fmol, 3 fmol, 1 fmol, 100 attomol, 10 attomol, and 1 attomol.
9. The method of aspect 6, comprising detecting the amount of the soluble, misfolded protein in the sample in a molar ratio to monomeric, folded protein comprised by the sample, the molar ratio being less than about one or more of: 1: 100, 1: 10,000, 1: 100,000, and 1: 1,000,000.
10. The method of aspect 1, comprising detecting the soluble, misfolded protein in the sample with a specificity of at least about one or more of: 80%, 81%, 82%, 83%, 84%, 85%, 86%, 87%, 88%, 89%, 90%, 91%, 92%, 93%, 94%, 95%, 96%, 97%, 98%, 99%, and 100%.
11. The method of aspect 1, comprising preparing the incubation mixture comprising the monomeric, folded protein at a concentration of one or more of: between about 1 nM and about 2 mM; between about 10 nM and about 200 µM; between about 100 nM and about 20 µM; between about 1 µM and about 10 µM; about 7 µM; and about 2 µM.
12. The method of aspect 1, comprising preparing the incubation mixture comprising a buffer composition including one or more of: Tris-HCL, PBS, MES, PIPES, MOPS, BES, TES, and HEPES, the incubation mixture comprising the buffer composition at a total concentration of between about 1 µm and about 1M.
13. The method of aspect 1, comprising preparing the incubation mixture comprising a salt composition at a total concentration of between about 1 µm and about 500 mM.
14. The method of aspect 13, the salt composition comprising one or more of: NaCl and KCI.
15. The method of aspect 1, comprising preparing or maintaining the incubation mixture with a pH of one or more of: between about 5 and about 9; between about 6 and about 8.5; between about 7 and about 8; and about 7.4.
16. The method of aspect 1, the incubating comprising incubating the incubation mixture at one or more of: between about 4 °C and about 60 °C; between about 4 °C and about 35 °C; between about 8 °C and about 50 °C; between about 12 °C and about 40 °C; between about 18 °C and about 30 °C; between about 18 °C and about 26 °C; and about 22 °C.
17. The method of aspect 1, the determining the presence of the soluble, misfolded protein in the sample comprising determining an amount of the soluble, misfolded protein in the sample compared to a control sample.
18. The method of aspect 1, the detecting the soluble, misfolded protein comprising one or more of: a Western Blot assay, a dot blot assay, an enzyme-linked immunosorbent assay (ELISA), a thioflavin T binding assay, a Congo Red binding assay, a sedimentation assay, electron microscopy, atomic force microscopy, surface plasmon resonance, and spectroscopy.
19. The method of aspect 18, the ELISA comprising a two-sided sandwich ELISA.
20. The method of aspect 18, the spectroscopy comprising one or more of: quasi-light scattering spectroscopy, multispectral ultraviolet spectroscopy, confocal dual-color fluorescence correlation spectroscopy, Fourier-transform infrared spectroscopy, capillary electrophoresis with spectroscopic detection, electron spin resonance spectroscopy, nuclear magnetic resonance spectroscopy, and Fluorescence Resonance Energy Transfer (FRET) spectroscopy.
21. The method of aspect 1, the detecting the soluble, misfolded protein comprising:
   contacting the incubation mixture with a protease; and
   detecting the soluble, misfolded protein using anti-misfolded protein antibodies in one or more of: a Western Blot assay, a dot blot assay, and an ELISA.
22. The method of aspect 1, further comprising providing the monomeric, folded protein in labeled form.
23. The method of aspect 22, the monomeric, folded protein in labeled form comprising one or more of: a covalently incorporated radioactive amino acid, a covalently incorporated, isotopically labeled amino acid, and a covalently incorporated fluorophore.
24. The method of aspect 22, the detecting the soluble, misfolded protein comprising detecting the monomeric, folded protein in labeled form as incorporated into the amplified portion of misfolded protein.
25. The method of aspect 1, the sample being taken from a subject, further comprising determining or diagnosing the presence of a protein misfolding disorder (PMD) in the subject according to the presence of the soluble, misfolded protein in the sample.
26. The method of aspect 25, the PMD comprising at least one of: an amyloidosis; a synucleinopathy; a triplet repeat disorder; and amyotrophic lateral sclerosis.
27. The method of aspect 25, the PMD comprising at least one of: Alzheimer's disease and systemic amyloidosis.
28. The method of aspect 25, the PMD comprising at least one of: Parkinson's disease, Lewy body dementia; multiple system atrophy; and synuclein-related neuroaxonal dystrophy.
29. The method of aspect 25, the PMD comprising Huntington's disease.
30. The method of aspect 1, the sample being taken from a subject, further comprising determining or diagnosing the presence of a PMD in the subject according to the presence of the soluble, misfolded protein in the sample compared to a control sample taken from a control subject.
31. The method of aspect 1, the detecting comprising detecting an amount of the soluble, misfolded protein in the sample, the sample being taken from a subject, further comprising determining or diagnosing the presence of a PMD in the subject by comparing the amount of the soluble, misfolded protein in the sample to a predetermined threshold amount.
32. The method of aspect 1, the sample being taken from a subject exhibiting no clinical signs of dementia according to cognitive testing, further comprising determining or diagnosing the presence of a PMD in the subject according to the presence of the soluble, misfolded protein in the sample.
33. The method of aspect 1, the sample being taken from a subject exhibiting no cortex plaques or tangles according to amyloid beta contrast imaging, further comprising determining or diagnosing the presence of a PMD in the subject according to the presence of the soluble, misfolded protein in the sample.
34. The method of aspect 1, the sample being taken from a subject exhibiting clinical signs of dementia according to cognitive testing, further comprising determining or diagnosing the presence of a PMD as a contributing factor to the clinical signs of dementia in the subject according to the presence of the soluble, misfolded protein in the sample.
35. The method of aspect 1, the sample being taken from a subject exhibiting no clinical signs of dementia according to cognitive testing, further comprising determining or diagnosing the presence of a PMD in the subject according to the presence of the soluble, misfolded protein in the sample.
36. The method of aspect 1, the sample comprising one or more of: amniotic fluid; bile; blood; cerebrospinal fluid; cerumen; skin; exudate; feces; gastric fluid; lymph; milk; mucus; mucosal membrane; peritoneal fluid; plasma; pleural fluid; pus; saliva; sebum; semen; sweat; synovial fluid; tears; and urine.
37. The method of aspect 1, further comprising obtaining the sample from a subject.
38. The method of aspect 37, the subject being one of a: human, mouse, rat, dog, cat, cattle, horse, deer, elk, sheep, goat, pig, and non-human primate.
39. The method of aspect 37, the subject being one or more of: at risk of a PMD, having the PMD, and under treatment for the PMD.
40. The method of aspect 1, the sample being taken from a subject, further comprising determining a progression or homeostasis of a PMD in the subject by comparing the amount of the soluble, misfolded protein in the sample to an amount of the soluble, misfolded protein in a comparison sample taken from the subject at a different time compared to the sample.
41. The method of aspect 1, the subject being treated with a PMD modulating therapy, further comprising:
   comparing the amount of the soluble, misfolded protein in the sample to an amount of the soluble, misfolded protein in a comparison sample, the sample and the comparison sample being taken from the subject at different times over a period of time under the PMD modulating therapy; and
   determining the subject is one of: responsive to the PMD modulating therapy according to a change in the soluble, misfolded protein over the period of time, or non-responsive to the PMD modulating therapy according to homeostasis of the soluble, misfolded protein over the period of time.
42. The method of aspect 41, further comprising treating the subject determined to be responsive to the PMD modulating therapy with the PMD modulating therapy.
43. The method of aspect 1, the sample being taken from a subject, the subject being treated with a PMD modulating therapy to inhibit the production of the monomeric, folded protein or to inhibit the aggregation of the soluble, misfolded protein.
44. The method of aspect 1, further comprising selectively concentrating the soluble, misfolded protein in one or more of the sample and the incubation mixture.
45. The method of aspect 44, the selectively concentrating the soluble, misfolded protein comprising pre-treating the sample prior to forming the incubation mixture.
46. The method of aspect 44, the selectively concentrating the soluble, misfolded protein comprising pre-treating the incubation mixture prior to incubating the incubation mixture.
47. The method of aspect 44, the selectively concentrating the soluble, misfolded protein comprising contacting one or more soluble, misfolded protein specific antibodies to the soluble, misfolded protein to form a captured soluble, misfolded protein.
48. The method of aspect 47, the one or more soluble, misfolded protein specific antibodies comprising one or more of: an antibody specific for an amino acid sequence of the soluble, misfolded protein and an antibody specific for a conformation of the soluble, misfolded protein.
49. The method of aspect 47, the one or more soluble, misfolded protein specific antibodies being coupled to a solid phase.
50. The method of aspect 49, the solid phase comprising one or more of a magnetic bead and a multiwell plate.
51. The method of aspect 49, the contacting the sample with the monomeric, folded protein to form the incubation mixture comprising contacting a molar excess of the monomeric, folded protein to the sample comprising the captured soluble, misfolded protein, the molar excess of the monomeric, folded protein being greater than a total molar amount of protein monomer included in the captured soluble, misfolded protein.
52. The method of aspect 49, the incubating the incubation mixture being effective to cause misfolding and/or aggregation of at least a portion of the monomeric, folded protein in the presence of the captured soluble, misfolded protein to form the amplified portion of misfolded protein.
53. The method of aspect 1, the protein aggregate comprising one or more of: the monomeric protein, the soluble, misfolded protein, and a captured form of the soluble, misfolded protein.
54. The method of aspect 1, the physically disrupting the incubation mixture comprising one or more of: sonication, stirring, shaking, freezing/thawing, laser irradiation, autoclave incubation, high pressure, and homogenization.
55. The method of aspect 1, the physically disrupting the incubation mixture comprising cyclic agitation.
56. The method of aspect 55, the cyclic agitation being conducted for one or more of: between about 50 rotations per minute (RPM) and 10,000 RPM, between about 200 RPM and about 2000 RPM, and at about 500 RPM.
57. The method of aspect 1, the physically disrupting the incubation mixture being conducted in each incubation cycle for one or more of: between about 5 seconds and about 10 minutes, between about 30 sec and about 1 minute, between about 45 sec and about 1 minute, and about 1 minute.
58. The method of aspect 1, the incubating the incubation mixture being independently conducted, in each incubation cycle for one or more of: between about 5 minutes and about 5 hours, between about 10 minutes and about 2 hours, between about 15 minutes and about 1 hour, and between about 25 minutes and about 45 minutes.
59. The method of aspect 1, each incubation cycle comprising independently incubating and physically disrupting the incubation mixture for one or more of: incubating between about 5 minutes and about 5 hours and physically disrupting between about 5 seconds and about 10 minutes; incubating between about 10 minutes and about 2 hours and physically disrupting between about 30 sec and about 1 minute; incubating between about 15 minutes and about 1 hour and physically disrupting between about 45 sec and about 1 minute; incubating between about 25 minutes and about 45 minutes and physically disrupting between about 45 sec and about 1 minute; and incubating about 1 minute and physically disrupting about 1 minute.
60. The method of aspect 1, the conducting the incubation cycle being repeated for one or more of: between about 2 times and about 1000 times, between about 5 times and about 500 times, between about 50 times and about 500 times, and between about 150 times and about 250 times.
61. The method of aspect 1, the incubating the incubation mixture being independently conducted, in each incubation cycle, at a temperature between about 15 °C and about 50 °C.
62. The method of aspect 1, the contacting the sample with the monomeric, folded protein to form the incubation mixture being conducted under physiological conditions.
63. The method of aspect 1, comprising contacting the sample with a molar excess of the monomeric, folded protein to form the incubation mixture, the molar excess being greater than a total molar amount of protein monomer included in the soluble, misfolded protein in the sample.
64. The method of aspect 1, comprising:
   contacting the sample with Thioflavin T and a molar excess of the monomeric, folded protein to form the incubation mixture, the molar excess being greater than an amount of protein monomer included in the soluble, misfolded protein in the sample;
   conducting the incubation cycle two or more times effective to form the amplified portion of misfolded protein, each incubation cycle comprising:
      incubating the incubation mixture effective to cause misfolding and/or aggregation of at least the portion of the monomeric, folded protein in the presence of the soluble, misfolded protein;
      shaking the incubation mixture effective to break up at least the portion of any protein aggregate present; and
   determining the presence of the soluble, misfolded protein in the sample by detecting a fluorescence of the Thioflavin T corresponding to the soluble, misfolded protein,
   the soluble, misfolded protein comprising one or more of: a soluble, misfolded monomer and a soluble, misfolded aggregate; and
   the amplified portion of misfolded protein comprising one or more of: an amplified portion of the soluble, misfolded monomer, an amplified portion of the soluble, misfolded aggregate, and an insoluble, misfolded aggregate.
65. The method of aspect 1, the monomeric, folded protein being produced by one of: chemical synthesis, recombinant production, and extraction from non-recombinant biological samples.
66. The method of aspect 1, the soluble, misfolded protein substantially being the soluble, misfolded aggregate.
67. The method of aspect 1, the amplified portion of misfolded protein substantially being one or more of: the amplified portion of the soluble, misfolded aggregate and the insoluble, misfolded aggregate.
68. The method of aspect 1, provided the sample excludes αS fibrils and tau fibrils.
69. A method for determining a presence of soluble, misfolded protein in a sample, comprising:
   capturing soluble, misfolded protein from the sample to form a captured soluble, misfolded protein;
   contacting the captured soluble, misfolded protein with a molar excess of a monomeric, folded protein corresponding to the soluble, misfolded protein to form an incubation mixture, the molar excess being greater than an amount of protein monomer included in the captured soluble, misfolded protein;
   conducting an incubation cycle two or more times effective to form an amplified portion of misfolded protein, each incubation cycle comprising:
      incubating the incubation mixture effective to cause misfolding and/or aggregation of at least a portion of the monomeric, folded protein in the presence of the captured soluble, misfolded protein;
      physically disrupting the incubation mixture effective to break up at least a portion of any protein aggregate present; and
   determining the presence of the soluble, misfolded protein in the sample by detecting at least a portion of the soluble, misfolded protein,
   the soluble, misfolded protein comprising one or more of: a soluble, misfolded monomer and a soluble, misfolded aggregate;
   the captured, soluble, misfolded protein comprising one or more of: a captured, soluble, misfolded monomer and a captured, soluble, misfolded aggregate; and
   the amplified portion of misfolded protein comprising one or more of: an amplified portion of the soluble, misfolded monomer, an amplified portion of the soluble, misfolded aggregate, and an insoluble, misfolded aggregate,
   provided that the monomeric, folded protein and the soluble, misfolded protein exclude prion protein (PrP) and isoforms thereof.
70. The method of aspect 69:
   further comprising contacting an indicator of the soluble, misfolded protein to the incubation mixture, the indicator of the soluble, misfolded protein being characterized by an indicating state in the presence of the soluble, misfolded protein and a non-indicating state in the absence of the soluble, misfolded protein; and
   wherein the determining the presence of the soluble, misfolded protein in the sample comprises detecting the indicating state of the indicator of the soluble, misfolded protein.
71. The method of aspect 69, the indicating state of the indicator and the non-indicating state of the indicator being characterized by a difference in fluorescence, the determining the presence of the soluble, misfolded protein in the sample comprising detecting the difference in fluorescence.
72. The method of aspect 69, comprising contacting a molar excess of the indicator of the soluble, misfolded protein to the incubation mixture, the molar excess being greater than a total molar amount of protein monomer included in the monomeric protein, the soluble, misfolded protein, and the captured soluble, misfolded protein in the incubation mixture.
73. The method of aspect 69, the indicator of the soluble, misfolded protein comprising one or more of: Thioflavin T, Congo Red, m-I-Stilbene, Chrysamine G, PIB, BF-227, X-34, TZDM, FDDNP, MeO-X-04, IMPY, NIAD-4, luminescent conjugated polythiophenes, a fluorescent protein, and derivatives thereof.
74. The method of aspect 69, the determining the presence of the soluble, misfolded protein in the sample comprising determining an amount of the soluble, misfolded protein in the sample.
75. The method of aspect 73, comprising detecting the amount of the soluble, misfolded protein in the sample at a sensitivity of at least about one or more of: 80%, 81%, 82%, 83%, 84%, 85%, 86%, 87%, 88%, 89%, 90%, 91%, 92%, 93%, 94%, 95%, 96%, 97%, 98%, 99%, and 100%.
76. The method of aspect 73, comprising detecting the amount of the soluble, misfolded protein in the sample at less than about one or more of: 100 nmol, 10 nmol, 1 nmol, 100 pmol, 10 pmol, 1 pmol, 100 fmol, 10 fmol, 3 fmol, 1 fmol, 100 attomol, 10 attomol, and 1 attomol.
77. The method of aspect 73, comprising detecting the amount of the soluble, misfolded protein in the sample in a molar ratio to monomeric, folded protein comprised by the sample, the molar ratio being less than about one or more of: 1: 100, 1: 10,000, 1: 100,000, and 1: 1,000,000.
78. The method of aspect 69, comprising detecting the soluble, misfolded protein in the sample with a specificity of at least about one or more of: 80%, 81%, 82%, 83%, 84%, 85%, 86%, 87%, 88%, 89%, 90%, 91%, 92%, 93%, 94%, 95%, 96%, 97%, 98%, 99%, and 100%.
79. The method of aspect 69, comprising preparing the incubation mixture comprising the monomeric, folded protein at a concentration of one or more of: between about 1 nM and about 2 mM; between about 10 nM and about 200 µM; between about 100 nM and about 20 µM; between about 1 µM and about 10 µM; and about 2 µM.
80. The method of aspect 69, comprising preparing the incubation mixture comprising a buffer composition including one or more of: Tris-HCL, PBS, MES, PIPES, MOPS, BES, TES, and HEPES, the incubation mixture comprising the buffer composition at a total concentration of between about 1 µm and about 1M.
81. The method of aspect 79, comprising preparing the incubation mixture comprising a salt composition at a total concentration of between about 1 µm and about 500 mM.
82. The method of aspect 80, the salt composition comprising one or more of: NaCl and KCI.
83. The method of aspect 69, comprising preparing or maintaining the incubation mixture with a pH of one or more of: between about 5 and about 9; between about 6 and about 8.5; between about 7 and about 8; and about 7.4.
84. The method of aspect 69, the incubating comprising incubating the incubation mixture at one or more of: between about 4 °C and about 60 °C; between about 4 °C and about 35 °C; between about 8 °C and about 50 °C; between about 12 °C and about 40 °C; between about 18 °C and about 30 °C; between about 18 °C and about 26 °C; and about 22 °C.
85. The method of aspect 69, the determining the presence of the soluble, misfolded protein in the sample comprising determining an amount of the soluble, misfolded protein in the sample compared to a control sample.
86. The method of aspect 69, the detecting the soluble, misfolded protein comprising one or more of: a Western Blot assay, a dot blot assay, an enzyme-linked immunosorbent assay (ELISA), a thioflavin T binding assay, a Congo Red binding assay, a sedimentation assay, electron microscopy, atomic force microscopy, surface plasmon resonance, and spectroscopy.
87. The method of aspect 85, the ELISA comprising a two-sided sandwich ELISA.
88. The method of aspect 85, the spectroscopy comprising one or more of: quasi-light scattering spectroscopy, multispectral ultraviolet spectroscopy, confocal dual-color fluorescence correlation spectroscopy, Fourier-transform infrared spectroscopy, capillary electrophoresis with spectroscopic detection, electron spin resonance spectroscopy, nuclear magnetic resonance spectroscopy, and Fluorescence Resonance Energy Transfer (FRET) spectroscopy.
89. The method of aspect 69, the detecting the soluble, misfolded protein comprising:
   contacting the incubation mixture with a protease; and
   detecting the soluble, misfolded protein using anti-misfolded protein antibodies in one or more of: a Western Blot assay, a dot blot assay, and an ELISA.
90. The method of aspect 69, further comprising providing the monomeric, folded protein in labeled form.
91. The method of aspect 89, the monomeric, folded protein in labeled form comprising one or more of: a covalently incorporated radioactive amino acid, a covalently incorporated, isotopically labeled amino acid, and a covalently incorporated fluorophore.
92. The method of aspect 89, the detecting the soluble, misfolded protein comprising detecting the monomeric, folded protein in labeled form as incorporated into the amplified portion of misfolded protein.
93. The method of aspect 69, the sample being taken from a subject, further comprising determining or diagnosing the presence of a PMD in the subject according to the presence of the soluble, misfolded protein in the sample.
94. The method of aspect 92, the PMD comprising at least one of: an amyloidosis; a synucleinopathy; a triplet repeat disorder; and amyotrophic lateral sclerosis.
95. The method of aspect 92, the PMD comprising at least one of: Alzheimer's disease and systemic amyloidosis.
96. The method of aspect 92, the PMD comprising at least one of: Parkinson's disease, Lewy body dementia; multiple system atrophy; and synuclein-related neuroaxonal dystrophy.
97. The method of aspect 92, the PMD comprising Huntington's disease.
98. The method of aspect 69, the sample being taken from a subject, further comprising determining or diagnosing the presence of a PMD in the subject according to the presence of the soluble, misfolded protein in the sample compared to a control sample taken from a control subject.
99. The method of aspect 69, the detecting comprising detecting an amount of the soluble, misfolded protein in the sample, the sample being taken from a subject, further comprising determining or diagnosing the presence of a PMD in the subject by comparing the amount of the soluble, misfolded protein in the sample to a predetermined threshold amount.
100. The method of aspect 69, the sample being taken from a subject exhibiting no clinical signs of dementia according to cognitive testing, further comprising determining or diagnosing the presence of a PMD in the subject according to the presence of the soluble, misfolded protein in the sample.
101. The method of aspect 69, the sample being taken from a subject exhibiting no cortex plaques or tangles according to amyloid beta contrast imaging, further comprising determining or diagnosing the presence of a PMD in the subject according to the presence of the soluble, misfolded protein in the sample.
102. The method of aspect 69, the sample being taken from a subject exhibiting clinical signs of dementia according to cognitive testing, further comprising determining or diagnosing the presence of a PMD as a contributing factor to the clinical signs of dementia in the subject according to the presence of the soluble, misfolded protein in the sample.
103. The method of aspect 69, the sample comprising one or more of: amniotic fluid; bile; blood; cerebrospinal fluid; cerumen; skin; exudate; feces; gastric fluid; lymph; milk; mucus; mucosal membrane; peritoneal fluid; plasma; pleural fluid; pus; saliva; sebum; semen; sweat; synovial fluid; tears; and urine.
104. The method of aspect 69, further comprising obtaining the sample from a subject.
105. The method of aspect 103, the subject being one of a: human, mouse, rat, dog, cat, cattle, horse, deer, elk, sheep, goat, pig, and non-human primate.
106. The method of aspect 103, the subject being one or more of: at risk of a PMD, having the PMD, and under treatment for the PMD.
107. The method of aspect 69, the sample being taken from a subject, further comprising determining or diagnosing a progression or homeostasis of a PMD in the subject by comparing the amount of the soluble, misfolded protein in the sample to an amount of the soluble, misfolded protein in a comparison sample taken from the subject at a different time compared to the sample.
108. The method of aspect 69, the subject being treated with a modulating therapy, further comprising:
   comparing the amount of the soluble, misfolded protein in the sample to an amount of the soluble, misfolded protein in a comparison sample, the sample and the comparison sample being taken from the subject at different times over a period of time under the PMD modulating therapy; and
   determining or diagnosing the subject is one of: responsive to the PMD modulating therapy according to a change in the soluble, misfolded protein over the period of time, or non-responsive to the PMD modulating therapy according to homeostasis of the soluble, misfolded protein over the period of time.
109. The method of aspect 107, further comprising treating the subject determined to be responsive to the PMD modulating therapy with the PMD modulating therapy.
110. The method of aspect 69, the capturing the soluble, misfolded protein from the sample to form a captured soluble, misfolded protein being conducted using one or more soluble, misfolded protein-specific antibodies, the one or more soluble, misfolded protein specific antibodies comprising one or more of: an antibody specific for an amino acid sequence of the soluble, misfolded protein and an antibody specific for a conformation of the soluble, misfolded protein.
111. The method of aspect 69, the capturing the soluble, misfolded protein from the sample to form a captured soluble, misfolded protein being conducted using one or more soluble, misfolded protein-specific antibodies, the one or more soluble, misfolded protein specific antibodies being coupled to a solid phase.
112. The method of aspect 110, the solid phase comprising one or more of a magnetic bead and a multiwell plate.
113. The method of aspect 69, the protein aggregate comprising one or more of: the monomeric protein, the soluble, misfolded protein, and a captured form of the soluble, misfolded protein.
114. The method of aspect 69, the physically disrupting the incubation mixture comprising one or more of: sonication, stirring, shaking, freezing/thawing, laser irradiation, autoclave incubation, high pressure, and homogenization.
115. The method of aspect 69, the physically disrupting the incubation mixture comprising cyclic agitation.
116. The method of aspect 114, the cyclic agitation being conducted for one or more of: between about 50 rotations per minute (RPM) and 10,000 RPM, between about 200 RPM and about 2000 RPM, and about 500 RPM.
117. The method of aspect 69, the physically disrupting the incubation mixture being conducted in each incubation cycle for one or more of: between about 5 seconds and about 10 minutes, between about 30 sec and about 1 minute, between about 45 sec and about 1 minute, and about 1 minute.
118. The method of aspect 69, the incubating the incubation mixture being independently conducted, in each incubation cycle for one or more of: between about 5 minutes and about 5 hours, between about 10 minutes and about 2 hours, between about 15 minutes and about 1 hour, and between about 25 minutes and about 45 minutes.
119. The method of aspect 69, each incubation cycle comprising independently incubating and physically disrupting the incubation mixture for one or more of: incubating between about 5 minutes and about 5 hours and physically disrupting between about 5 seconds and about 10 minutes; incubating between about 10 minutes and about 2 hours and physically disrupting between about 30 sec and about 1 minute; incubating between about 15 minutes and about 1 hour and physically disrupting between about 45 sec and about 1 minute; incubating between about 25 minutes and about 45 minutes and physically disrupting between about 45 sec and about 1 minute; and incubating about 1 minute and physically disrupting about 1 minute.
120. The method of aspect 69, the conducting the incubation cycle being repeated for one or more of: between about 2 times and about 1000 times, between about 5 times and about 500 times, between about 50 times and about 500 times, and between about 150 times and about 250 times.
121. The method of aspect 69, the incubating the incubation mixture being independently conducted, in each incubation cycle, at a temperature between about 15 °C and about 50 °C.
122. The method of aspect 69, the contacting the sample with the monomeric, folded protein to form the incubation mixture being conducted under physiological conditions.
123. The method of aspect 69, comprising contacting the sample with a molar excess of the monomeric, folded protein to form the incubation mixture, the molar excess being greater than a total molar amount of protein monomer included in the soluble, misfolded protein in the sample.
124. The method of aspect 69, the monomeric, folded protein being produced by one of: chemical synthesis, recombinant production, and extraction from non-recombinant biological samples.
125. The method of aspect 69, the soluble, misfolded protein substantially being the soluble, misfolded aggregate.
126. The method of aspect 69, the amplified portion of misfolded protein substantially being one or more of: the amplified portion of the soluble, misfolded aggregate and the insoluble, misfolded aggregate.
127. The method of aspect 69, provided the sample excludes αS fibrils and tau fibrils.
128. A kit for determining a presence of soluble, misfolded protein in a sample, comprising:
   one or more of a known amount of a monomeric, folded protein corresponding to the soluble, misfolded protein and a known amount of an indicator of the soluble, misfolded protein;
   instructions, the instructions directing a user to:
      contact the sample with one or more of the known amount of the monomeric, folded protein and the known amount of the indicator of the soluble, misfolded protein to form an incubation mixture;
      conduct an incubation cycle two or more times effective to form an amplified portion of misfolded protein, each incubation cycle comprising:
         incubating the incubation mixture effective to cause misfolding and/or aggregation of at least a portion of the monomeric, folded protein in the presence of the soluble, misfolded protein to form the amplified portion of misfolded protein;
         physically disrupting the incubation mixture effective to break up at least a portion of any protein aggregate present; and
      determine the presence of the soluble, misfolded protein in the sample by detecting the soluble, misfolded protein,
   the soluble, misfolded protein comprising one or more of: a soluble, misfolded monomer and a soluble, misfolded aggregate; and
   the amplified portion of misfolded protein comprising one or more of: an amplified portion of the soluble, misfolded monomer, an amplified portion of the soluble, misfolded aggregate, and an insoluble, misfolded aggregate,
   provided that the monomeric, folded protein and the soluble, misfolded protein exclude prion protein (PrP) and isoforms thereof.
129. The kit of aspect 128, comprising the known amount of the monomeric, folded protein and the known amount of the indicator of the soluble, misfolded protein.
130. The kit of aspect 128, further comprising one or more of: a multiwall microtitre plate; a microfluidic plate; a shaking apparatus; and an incubating apparatus.
131. The kit of aspect 128, an indicating state and a non-indicating state of the indicator of the soluble, misfolded protein being characterized by a difference in fluorescence, and the instructions directing the user to determine the presence of the soluble, misfolded protein in the sample by fluorescence spectroscopy.
132. The kit of aspect 128, the indicator of the soluble, misfolded protein comprising one or more of: Thioflavin T, Congo Red, m-I-Stilbene, Chrysamine G, PIB, BF-227, X-34, TZDM, FDDNP, MeO-X-04, IMPY, NIAD-4, luminescent conjugated polythiophenes, a fluorescent protein, and derivatives thereof.
133. The kit of aspect 128, the instructions directing the user to determine the presence of the soluble, misfolded protein in the sample comprising directions to determine an amount of the soluble, misfolded protein in the sample.
134. The kit of aspect 128, the instructions directing the user to detect the soluble, misfolded protein comprising directions to conduct one or more of: a Western Blot assay, a dot blot assay, an enzyme-linked immunosorbent assay (ELISA), a thioflavin T binding assay, a Congo Red binding assay, a sedimentation assay, electron microscopy, atomic force microscopy, surface plasmon resonance, and spectroscopy.
135. The kit of aspect 128, the instructions directing the user to detect the soluble, misfolded protein comprising directions to:
   contact the incubation mixture with a protease; and
   detect the soluble, misfolded protein using anti-misfolded protein antibodies in one or more of: a Western Blot assay, a dot blot assay, and an ELISA.
136. The kit of aspect 128, the monomeric, folded protein comprising one or more of a covalently incorporated radioactive amino acid, a covalently incorporated, isotopically labeled amino acid, and a covalently incorporated fluorophore.
137. The kit of aspect 128, the instructions further comprising directing the user to take the sample from a subject.
138. The kit of aspect 137, the instructions further comprising directing the user to determine the presence of a PMD in the subject according to the presence of the soluble, misfolded protein in the sample.
139. The kit of aspect 138, the PMD comprising at least one of: an amyloidosis; a synucleinopathy; a triplet repeat disorder; and amyotrophic lateral sclerosis.
140. The kit of aspect 138, the PMD comprising at least one of: Alzheimer's disease and systemic amyloidosis.
141. The kit of aspect 138, the PMD comprising at least one of: Parkinson's disease, Lewy body dementia; multiple system atrophy; and synuclein-related neuroaxonal dystrophy.
142. The kit of aspect 138, the PMD comprising Huntington's disease.
143. The kit of aspect 138, providing detection of the amount of the soluble, misfolded protein in the sample with a sensitivity of at least about one or more of: 80%, 81%, 82%, 83%, 84%, 85%, 86%, 87%, 88%, 89%, 90%, 91%, 92%, 93%, 94%, 95%, 96%, 97%, 98%, 99%, and 100%.
144. The kit of aspect 138, providing detection of the amount of the soluble, misfolded protein in the sample at less than about one or more of: 100 nmol, 10 nmol, 1 nmol, 100 pmol, 10 pmol, 1 pmol, 100 fmol, 10 fmol, 3 fmol, 1 fmol, 100 attomol, 10 attomol, and 1 attomol.
145. The kit of aspect 138, providing detection of the amount of the soluble, misfolded protein in the sample in a molar ratio to monomeric, folded protein comprised by the sample, the molar ratio being less than about one or more of 1: 100, 1: 10,000, 1: 100,000, and 1: 1,000,000.
146. The kit of aspect 128, providing detection of the soluble, misfolded protein in the sample with a specificity of at least about one or more of: 80%, 81%, 82%, 83%, 84%, 85%, 86%, 87%, 88%, 89%, 90%, 91%, 92%, 93%, 94%, 95%, 96%, 97%, 98%, 99%, and 100%.
147. The kit of aspect 128, the instructions further comprising directions to the user to prepare the incubation mixture comprising the monomeric, folded protein in a concentration range of one or more of: between about 1 nM and about 2 mM; between about 10 nM and about 200 µM; between about 100 nM and about 20 µM; and between about 1 µM and about 10 µM.
148. The kit of aspect 128, further comprising a buffer composition comprising one or more of: Tris-HCL, PBS, MES, PIPES, MOPS, BES, TES, and HEPES; and the instructions further comprising directions to the user to prepare the incubation mixture comprising the buffer composition at a total concentration of between about 1 µm and about 1M.
149. The kit of aspect 148, further comprising a salt composition, the instructions further comprising directions to the user to prepare the incubation mixture comprising the salt composition at a total concentration of between about 1 µm and about 500 mM.
150. The kit of aspect 149, the salt composition comprising one or more of: NaCl and KCI.
151. The kit of aspect 128, the instructions further comprising directions to the user to prepare or maintain the incubation mixture in a pH range of one or more of: between about 5 and about 9; between about 6 and about 8.5; between about 7 and about 8; and about 7.4.
152. The kit of aspect 128, the instructions further comprising directions to the user to incubate the incubation mixture at one or more of: between about 4 °C and about 60 °C; between about 4 °C and about 35 °C; between about 8 °C and about 50 °C; between about 12 °C and about 40 °C; between about 18 °C and about 30 °C; between about 18 °C and about 26 °C; and about 22 °C.
153. The kit of aspect 128, the instructions further comprising directing the user to determine the presence of a PMD in the subject according to the presence of the soluble, misfolded protein in the sample compared to a control sample taken from a control subject.
154. The kit of aspect 128, the instructions further comprising directing the user to determine the presence of a PMD in the subject by comparing the amount of the soluble, misfolded protein in the sample to a predetermined threshold amount.
155. The kit of aspect 128, the instructions comprising directing the user to obtain the sample comprising one or more of: amniotic fluid; bile; blood; cerebrospinal fluid; cerumen; skin; exudate; feces; gastric fluid; lymph; milk; mucus; mucosal membrane; peritoneal fluid; plasma; pleural fluid; pus; saliva; sebum; semen; sweat; synovial fluid; tears; and urine.
156. The kit of aspect 128, the instructions further comprising directing the user to determine a progression or homeostasis of PMD in the subject by comparing the amount of the soluble, misfolded protein in the sample to an amount of the soluble, misfolded protein in a comparison sample taken from the subject at a different time compared to the sample.
157. The kit of aspect 128, the instructions further comprising directing the user to selectively concentrate the soluble, misfolded protein in one or more of the sample and the incubation mixture.
158. The kit of aspect 128, comprising one or more soluble, misfolded protein specific antibodies.
159. The kit of aspect 158, the one or more soluble, misfolded protein specific antibodies comprising one or more of: an antibody specific for an amino acid sequence of the monomeric, folded protein and an antibody specific for a conformation of the soluble, misfolded protein.
160. The kit of aspect 159, the instructions further comprising directing the user to selectively concentrate the soluble, misfolded protein by contacting the one or more soluble, misfolded protein specific antibodies to the soluble, misfolded protein to form a captured soluble, misfolded protein.
161. The kit of aspect 159, the one or more soluble, misfolded protein specific antibodies being provided coupled to a solid phase.
162. The kit of aspect 159, the solid phase comprising one or more of a magnetic bead and a multiwell plate.
163. The kit of aspect 128, the instructions for physically disrupting the incubation mixture comprising directions to a user to employ one or more of: sonication, stirring, shaking, freezing/thawing, laser irradiation, autoclave incubation, high pressure, and homogenization.
164. The kit of aspect 128, the instructions for physically disrupting the incubation mixture comprising directions to a user to conduct cyclic agitation for one or more of: between about 50 rotations per minute (RPM) and 10,000 RPM, between about 200 RPM and about 2000 RPM, and about 500 RPM.
165. The kit of aspect 128, the instructions for physically disrupting the incubation mixture comprising directions to a user to conduct the physical disruption in each incubation cycle for one or more of: between about 5 seconds and about 10 minutes, between about 30 sec and about 1 minute, between about 45 sec and about 1 minute, and about 1 minute.
166. The kit of aspect 128, the instructions for incubating the incubation mixture comprising directions to a user to incubating the incubation mixture in each incubation cycle for one or more of: between about 5 minutes and about 5 hours, between about 10 minutes and about 2 hours, between about 15 minutes and about 1 hour, and between about 25 minutes and about 45 minutes.
167. The kit of aspect 128, the instructions for each incubation cycle comprising directions to a user for incubating and physically disrupting the incubation mixture for one or more of: incubating between about 5 minutes and about 5 hours and physically disrupting between about 5 seconds and about 10 minutes; incubating between about 10 minutes and about 2 hours and physically disrupting between about 30 sec and about 1 minute; incubating between about 15 minutes and about 1 hour and physically disrupting between about 45 sec and about 1 minute; incubating between about 25 minutes and about 45 minutes and physically disrupting between about 45 sec and about 1 minute; and incubating about 1 minute and physically disrupting about 1 minute.
168. The kit of aspect 128, the instructions for conducting the incubation cycle comprising directions to a user to conduct the incubation cycle for one or more of: between about 2 times and about 1000 times, between about 5 times and about 500 times, between about 50 times and about 500 times, and between about 150 times and about 250 times.
169. The kit of aspect 128, the instructions for conducting the incubation cycle comprising directions to a user to incubate at a temperature between about 15 °C and about 50 °C.
170. The kit of aspect 128, the monomeric, folded protein being produced by one of: chemical synthesis, recombinant production, and extraction from non-recombinant biological samples.
171. The kit of aspect 128, the soluble, misfolded protein substantially being the soluble, misfolded aggregate.
172. The kit of aspect 128, the amplified portion of misfolded protein substantially being one or more of: the amplified portion of the soluble, misfolded aggregate and the insoluble, misfolded aggregate.
173. The kit of aspect 128, the instructions directing the user to provide the sample excluding αS fibrils and tau fibrils.

## Claims

1. An in vitro method of diagnosing alpha-synucleinopathies, the method comprising:
(A) providing an incubation mixture, the incubation mixture comprising:
(1) a monomeric, folded protein comprising alpha-synuclein not associated with alpha-synuclein seeds, in a concentration range of about 10 nM to about 200 µM;
(2) a buffer having a pH between 6 and 7.4;
(3) a salt composition comprising one or more of NaCl or KCI, said salt composition having a total concentration of 500 mM +-10%;
(4) thioflavin T (ThT); and
(5) the sample;
wherein said monomeric, folded protein comprising alpha-synuclein is in molar excess to thioflavin;
(B) conducting out an incubation cycle two or more times effective to form an amplified portion of misfolded protein, comprising: incubating the incubation mixture at a temperature between 15 °C and 50 °C to cause aggregation of at least a portion of the monomeric, folded protein in presence of the soluble, aggregated misfolded protein and shaking the incubation mixture effective to break up at least the portion of any protein aggregate present; and
(C) determining the presence of the soluble, aggregated misfolded protein in the sample by detecting fluorescence of the ThT, wherein the presence of the soluble, aggregated misfolded protein comprising alpha-synuclein is indicative of an alpha-synucleinopathy.

2. The method of claim 1, wherein the monomeric, folded protein comprising alpha-synuclein protein is produced by one of: chemical synthesis, recombinant production, or extraction from non-recombinant biological fluids.

3. The method of claim 1 or 2, wherein the step of incubating the incubation mixture comprises subjecting the incubation mixture to cyclic agitation.

4. The method of any one of claims 1-3, wherein the step of illuminating the incubated mixture with a wavelength of light that excites the ThT comprises illuminating at about 435 nm, and monitoring ThT fluorescence at about 485 nm.

5. The method of any one of claims 1-4, wherein at least one of the step of incubating the incubation mixture with agitation cycles, the step of determining is performed using a microplate reader.

6. The method of any one of claims 1-5, wherein the biological fluid comprises one or more of: amniotic fluid; bile; blood; cerebrospinal fluid; cerumen; skin; exudate; feces; gastric fluid; lymph; milk; mucus; mucosal membrane; peritoneal fluid; blood plasma; pleural fluid; pus; saliva; sebum; semen; sweat; synovial fluid; tears; and urine.

7. The method of any one of claims 1-6, wherein the synucleinopathy comprises at least one of Parkinson's disease, Lewy body dementia, multiple system atrophy, and synuclein-related neuroaxonal dystrophy.

8. A kit for detecting the presence of soluble, aggregated misfolded protein comprising alpha-synuclein aggregates in a sample comprising a biological fluid, the kit comprising:
a known amount of a monomeric, folded protein comprising alpha-synuclein not associated with alpha-synuclein seeds;
a known amount of an indicator of the soluble, aggregated misfolded protein comprising one or more of Thioflavin T, Congo Red, m-I-Stilbene, Chrysamine G, PIB, BF-227, X-34, TZDM, FDDNP, MeO-X-04, IMPY, NIAD-4, luminescent conjugated polythiophenes, a fluorescent protein, and derivatives thereof;
a buffer composition comprising one or more of Tris-HCL, PBS, MES, PIPES, MOPS, BES, TES, and HEPES; and
a salt composition comprising one or more of NaCl and KCI.

9. The kit of claim 8, wherein the known amount of a monomeric, folded protein comprising alpha-synuclein not associated with alpha-synuclein seeds is a concentration of about 10 nM to about 200 µM.

10. The kit of claim 8 or 9, wherein the salt composition is at a total concentration of about 500 mM.

11. The kit of claim 8, wherein the buffer has a pH between 6 and 7.4.

12. The kit of claim 8, further comprising a solid phase comprising one or more solid phase of a bead and a multiwell plate.

13. The kit of claim 12, further comprising one or more soluble, misfolded protein specific antibodies.

14. The kit of claim 13, the one or more soluble, misfolded protein specific antibodies being provided coupled to the solid phase.

15. The kit of any of claims 8-14, wherein the, where present, is a bead, and the kit further comprises one or more of: a multiwall microtitre plate; a microfluidic plate; a shaking apparatus; and an incubating apparatus.
